# EUROPEAN PATENT SPECIFICATION

(11) **EP 2 253 712 B1**
(45) Date of publication and mention of the grant of the patent: **14.01.2015**
(21) Application number: 09718220.8
(22) Date of filing: 03.03.2009
(51) Int. Cl.: C12Q 1/68, C12N 15/09, G01N 33/569, C12Q 1/06

(54) **METHOD OF DISTINGUISHING INFLAMMATORY PATHOGEN CAUSING ACUTE RESPIRATORY INFECTION**
VERFAHREN ZUR ERKENNUNG EINES ENTZÜNDUNGSERREGERS ALS VERURSACHER VON AKUTER ATEMWEGSINFEKTION
PROCÉDÉ POUR DISTINGUER UN PATHOGÈNE INFLAMMATOIRE PROVOQUANT UNE INFECTION RESPIRATOIRE AIGUË

(30) Priority: 03.03.2008 JP 2008052399
(43) Date of publication of application: 24.11.2010
(73) Proprietor: Saitama Medical University, Iruma-gun, Saitama 350-0495 (JP)
(72) Inventor: HIRAMA, Takashi, Iruma-gun Saitama 350-0495 (JP); HAGIWARA, Koichi, Iruma-gun Saitama 350-0495 (JP)
(74) Representative: Cabinet Plasseraud
(86) International application number: PCT/JP2009/053976
(87) International publication number: WO 2009/110473

(56) References cited:
- WO-A1-2007/075026
- KAIS ET AL: "Quantitative detection of Streptococcus pneumoniae, Haemophilus influenzae, and Moraxella catarrhalis in lower respiratory tract samples by real-time PCR", DIAGNOSTIC MICROBIOLOGY AND INFECTIOUS DISEASES, ELSEVIER SCIENCE PUBLISHING CO., AMSTERDAM, NL, vol. 55, no. 3, 1 July 2006 (2006-07-01), pages 169-178, XP005517856, ISSN: 0732-8893, DOI: 10.1016/J.DIAGMICROBIO.2006.01.007
- YANG S ET AL: "PCR-based diagnostics for infectious diseases: uses, limitations, and future applications in acute-care settings", LANCET INFECTIOUS DISEASES, ELSEVIER LTD, US, vol. 4, no. 6, 1 June 2004 (2004-06-01), pages 337-348, XP004808604, ISSN: 1473-3099, DOI: 10.1016/S1473-3099(04)01044-8
- TAKASHI HIRAMA ET AL: "Prediction of the Pathogens That Are the Cause of Pneumonia by the Battlefield Hypothesis", PLOS ONE, vol. 6, no. 9, 1 January 2011 (2011-01-01) , page e24474, XP55042450, ISSN: 1932-6203, DOI: 10.1371/journal.pone.0024474
- KAIS, M. ET AL.: 'Quantitative detection of Streptococcus pneumoniae, Haemophilus influenzae, and Moraxella catarrhalis in lower respiratory tract samples by real-time PCR' DIAGN MICROBIOL INFECT DIS vol. 55, no. 3, July 2006, pages 169 - 178, XP005517856
- LODE, H. ET AL.: 'Diagnostic problems in lower respiratory tract infections' J ANTIMICROB CHEMOTHER vol. 32, no. SUP.A, 1993, pages 29 - 37, XP008140629
- MUSHER D. M.: 'How contagious are common respiratory tract infections?' N ENGL J MED. vol. 348, no. 13, 2003, pages 1256 - 1266, XP008140620
- HIRAMA T. ET AL.: 'Hyojunka Hanteiryo Real-time PCR o Mochiita Sokatsuteki Byogentai Kensaku ni yoru Kokyuki Kansensho Kienkin Shindan' DAI 48 KAI THE JAPANESE RESPIRATORY SOCIETY GAKUJUTSU KOENKAI PROGRAM 10 May 2008, page 158, XP008140630
- HIRAMA T. ET AL.: 'Hyojunka Hanteiryo Real-time PCR o Mochiite no Kokyuki Kansensho no Kien Byogentai no Doteiho-Atarashii Haien Kensa no Kokoromi' JOURNAL OF THE JAPANESE SOCIETY FOR CLINICAL MICROBIOLOGY vol. 18, no. 4, 25 December 2008, page 125, XP008142846
- TAKASHI HIRAMA ET AL.: 'Hyojunka Hanteiryo Real-time PCR o Mochiite no Kokyuki Kansensho no Kien Byogentai Doteiho-Atarashii Haien Kensa no Kokoromi' DAI 57 KAI JAPANESE ASSOCIATION FOR INFECTIOUS DISEASES HIGASHI NIPPON CHIHOKAI SOKAI · DAI 55 KAI HIGASHINIHON BRANCH OF JAPAN SOCIETY OF CHEMOTHERAPY SOKAI GODOKAI PROGRAM· KOEN YOKOSHU 01 October 2008, page 137

### SEQUENCE LISTING

<110> Saitama Medical University
<120> Method of Discriminating Inflammatory Pathogen of Acute Respiratory
   Infection
<130> FP09-0094-00
<160> 64
<170> PatentIn version 3.1
<210> 1
   <211> 21
   <212> DNA
   <213> Homo sapiens
<400> 1
   gcagtgccta cgtctaagct g 21
<210> 2
   <211> 23
   <212> DNA
   <213> Homo sapiens
<400> 2
   tagatgtagt agagcggcac ctc 23
<210> 3
   <211> 24
   <212> DNA
   <213> Streptococcus pneumoniae
<400> 3
   ctaaggcttg ggacagaaat gggc 24
<210> 4
   <211> 24
   <212> DNA
   <213> Streptococcus pneumoniae
<400> 4
   ccgcttacgc actagtggca aatc 24
<210> 5
   <211> 26
   <212> DNA
   <213> Haemophilus influenzae
<400> 5
   ttgacatcct aagaagagct cagaga 26
<210> 6
   <211> 26
   <212> DNA
   <213> Haemophilus influenzae
<400> 6
   cttccctctg tatacgccat tgtagc 26
<210> 7
   <211> 26
   <212> DNA
   <213> Moraxella catarrhalis
<400> 7
   cgtgcgtgtt gaccgttttg acttta 26
<210> 8
   <211> 26
   <212> DNA
   <213> Moraxella catarrhalis
<400> 8
   cacgctgcca aaaataactg ccaaag 26
<210> 9
   <211> 22
   <212> DNA
   <213> Pseudomonas sp.
<400> 9
   gacgggtgag taatgcctag ga 22
<210> 10
   <211> 24
   <212> DNA
   <213> Pseudomonas sp.
<400> 10
   ccactggtgt tccttcctat atct 24
<210> 11
   <211> 23
   <212> DNA
   <213> Klebsiella pneumoniae
<400> 11
   tgaagtatga ctccactcac ggt 23
<210> 12
   <211> 24
   <212> DNA
   <213> Klebsiella pneumoniae
<400> 12
   cttcagaagc ggctttgatg gctt 24
<210> 13
   <211> 26
   <212> DNA
   <213> Stenotrophomonas maltophilia
<400> 13
   ggaagcgaac ctggtgaact gaaata 26
<210> 14
   <211> 26
   <212> DNA
   <213> Stenotrophomonas maltophilia
<400> 14
   cgcacacggt ttcaggttct atttca 26
<210> 15
   <211> 24
   <212> DNA
   <213> Staphylococcus aureus
<400> 15
   tggccactat gagttaaagc ttgc 24
<210> 16
   <211> 24
   <212> DNA
   <213> Staphylococcus aureus
<400> 16
   tcataatcaa tcactggacc gcga 24
<210> 17
   <211> 24
   <212> DNA
   <213> Staphylococcus aureus
<400> 17
   aactacggta acattgatcg caac 24
<210> 18
   <211> 24
   <212> DNA
   <213> Staphylococcus aureus
<400> 18
   ctttggtctt tctgcattcc tgga 24
<210> 19
   <211> 27
   <212> DNA
   <213> Metallo-beta-lactamase
<400> 19
   ggcagyattt cctctcattt tcatagc 27
<210> 20
   <211> 27
   <212> DNA
   <213> Metallo-beta-lactamase
<400> 20
   aatttgtrgc ttgaacctta ccgtctt 27
<210> 21
   <211> 27
   <212> DNA
   <213> Mycoplasma pneumoniae
<400> 21
   agtaatactt tagaggcgaa cgggtga 27
<210> 22
   <211> 27
   <212> DNA
   <213> Mycoplasma pneumoniae
<400> 22
   tctacttctc agcatagcta cacgtca 27
<210> 23
   <211> 24
   <212> DNA
   <213> Legionella pneumophila
<400> 23
   taaccgaaca gcaaatgaaa gacg 24
<210> 24
   <211> 24
   <212> DNA
   <213> Legionella pneumophila
<400> 24
   aaaacggtac catcaatcag acga 24
<210> 25
   <211> 24
   <212> DNA
   <213> Legionella sp.
<400> 25
   aggctaatct taaagcgcca ggcc 24
<210> 26
   <211> 26
   <212> DNA
   <213> Legionella sp.
<400> 26
   gcatgcttaa cacatgcaag tcgaac 26
<210> 27
   <211> 25
   <212> DNA
   <213> Chlamydophila pneumoniae
<400> 27
   gcaaccacgg tagcaacaca aatta 25
<210> 28
   <211> 25
   <212> DNA
   <213> Chlamydophila pneumoniae
<400> 28
   aattgagcga cgttttgttg catct 25
<210> 29
   <211> 28
   <212> DNA
   <213> Chlamydophila psittaci
<400> 29
   gtatgttcat gcttaaggct gttttcac 28
<210> 30
   <211> 28
   <212> DNA
   <213> Chlamydophila psittaci
<400> 30
   tcccacatag tgccatcgat taataaac 28
<210> 31
   <211> 26
   <212> DNA
   <213> Nocardia sp.
<400> 31
   ccttcgggtt gtaaacctct ttcgac 26
<210> 32
   <211> 24
   <212> DNA
   <213> Nocardia sp.
<400> 32
   ttggggttga gccccaagtt ttca 24
<210> 33
   <211> 26
   <212> DNA
   <213> Pneumocystis jiroveci
<400> 33
   gtgtacgttg caaagtactc agaaga 26
<210> 34
   <211> 20
   <212> DNA
   <213> Pneumocystis jiroveci
<400> 34
   gatggctgtt tccaagccca 20
<210> 35
   <211> 21
   <212> DNA
   <213> Mycobacterium tuberculosis
<400> 35
   atccgctgcc agtcgtcttc c 21
<210> 36
   <211> 21
   <212> DNA
   <213> Mycobacterium tuberculosis
<400> 36
   ctcgcgagtc taggccagca t 21
<210> 37
   <211> 24
   <212> DNA
   <213> Mycobacterium intracellulare, Mycobacterium avium
<400> 37
   agcaccacga aaagcactcc aatt 24
<210> 38
   <211> 24
   <212> DNA
   <213> Mycobacterium intracellulare
<400> 38
   cgaacgcatc agccctaagg acta 24
<210> 39
   <211> 25
   <212> DNA
   <213> Mycobacterium avium
<400> 39
   aattacacat ttcgatgaac gccgg 25
<210> 40
   <211> 23
   <212> DNA
   <213> Mycobacterium kansasii
<400> 40
   acccgtgtga tgagtgcaaa ggc 23
<210> 41
   <211> 24
   <212> DNA
   <213> Mycobacterium kansasii
<400> 41
   gtaaagctga ccggaactgt gacg 24
<210> 42
   <211> 23
   <212> DNA
   <213> Homo sapiens
<400> 42
   cgagatgcag gctcagcacc ctc 23
<210> 43
   <211> 26
   <212> DNA
   <213> Streptococcus pneumoniae
<400> 43
   agggaatgtt cgcaatctct ctgtca 26
<210> 44
   <211> 22
   <212> DNA
   <213> Haemophilus influenzae
<400> 44
   atggctgtcg tcagctcgtg tt 22
<210> 45
   <211> 26
   <212> DNA
   <213> Moraxella catarrhalis
<400> 45
   cagcggtaac ctaatctatg ccactc 26
<210> 46
   <211> 22
   <212> DNA
   <213> Pseudomonas sp.
<400> 46
   agtgggggat cttcggacct ca 22
<210> 47
   <211> 22
   <212> DNA
   <213> Klebsiella pneumoniae
<400> 47
   ccggtatctt cctgaccgac ga 22
<210> 48
   <211> 24
   <212> DNA
   <213> Stenotrophomonas maltophilia
<400> 48
   accgaccgat agtgaaccag tacc 24
<210> 49
   <211> 26
   <212> DNA
   <213> Staphylococcus aureus
<400> 49
   cgaggtcatt gcagcttgct tactta 26
<210> 50
   <211> 27
   <212> DNA
   <213> Staphylococcus aureus
<400> 50
   agatggtatg tggaagttag attggga 27
<210> 51
   <211> 26
   <212> DNA
   <213> Metallo-beta-lactamase
<400> 51
   attctcgatc tatccccacg tatgca 26
<210> 52
   <211> 22
   <212> DNA
   <213> Mycoplasma pneumoniae
<400> 52
   accaactagc tgatatggcg ca 22
<210> 53
   <211> 23
   <212> DNA
   <213> Legionella pneumophila
<400> 53
   tgatggcaaa gcgtactgct gaa 23
<210> 54
   <211> 26
   <212> DNA
   <213> Legionella sp.
<400> 54
   catattccta cgcgttactc acccgt 26
<210> 55
   <211> 26
   <212> DNA
   <213> Chlamydophila pneumoniae
<400> 55
   agcggctgtc aaatctggaa taaaag 26
<210> 56
   <211> 28
   <212> DNA
   <213> Chlamydophila psittaci
<400> 56
   ccagaagagc aaattagaat agcgagca 28
<210> 57
   <211> 24
   <212> DNA
   <213> Nocardia sp.
<400> 57
   aagaagcacc ggccaactac gtgc 24
<210> 58
   <211> 26
   <212> DNA
   <213> Pneumocystis jiroveci
<400> 58
   ctaggatata gctggttttc tgcgaa 26
<210> 59
   <211> 25
   <212> DNA
   <213> Mycobacterium tuberculosis
<400> 59
   ccggacaaca ggtatcgata gcgcc 25
<210> 60
   <211> 24
   <212> DNA
   <213> Mycobacterium intracellulare, Mycobacterium avium
<400> 60
   cctgagacaa cactcggtcg atcc 24
<210> 61
   <211> 22
   <212> DNA
   <213> Mycobacterium kansasii
<400> 61
   aggacggaca gcggatcaga ct 22
<210> 62
   <211> 22
   <212> DNA
   <213> Mycobacterium avium
<400> 62
   caagtcgaac ggaaaggcct ct 22
<210> 63
   <211> 22
   <212> DNA
   <213> Mycobacterium avium
<400> 63
   gccgtatctc agtcccagtg tg 22
<210> 64
   <211> 23
   <212> DNA
   <213> Mycobacterium avium
<400> 64
   taccggatag gacctcaaga cgc 23

## Description

### Technical Field

The present invention relates to a method of distinguishing a respiratory pathogen causing acute respiratory infection.

### Background Art

Acute respiratory infection such as pneumonia is classified into bacterial (also including those caused by acid-fast bacteria such as *Mycobacterium tuberculosis*), fungal, and viral infections, and the like, according to the type of the respiratory pathogen. Moreover, pneumonia is classified in clinical practice into community-acquired pneumonia, health-care associated pneumonia (which refers mainly to aspiration pneumonia), and hospital-acquired pneumonia (which includes pneumonia caused under specific conditions, such as ventilator-associated pneumonia and opportunistic pneumonia) according to where it occurs. The epidemiologic characteristics such as the type or frequency of the respiratory pathogen have been confirmed to differ depending on the place where it occurs. Moreover, since it is very important for acute respiratory infection to identify the respiratory pathogen thereof for confirming the diagnosis and deciding a therapeutic course, a more highly reliable method of testing pneumonia pathogens has been desired.

Gram staining of smear, culture test, and serodiagnosis are known as methods of testing respiratory pathogens causing acute respiratory infection. Moreover, a urinary antigen detection method (Non-Patent Document 1) and genetic testing (Patent Document 1 and Non-Patent Document 2) targeting *Legionella* or *Streptococcus pneumoniae* have been developed.
Patent Document 1: Japanese Patent Laid-Open No. 2005-110545
Non-Patent Document 1: Clin. Infect. Dis. 44: S27-72 (2007)
Non-Patent Document 2: Nippon Rinsho 65 Suppl 2: 199-207 (2007)
Non-Patent Document 3: N. Engl. J. Med. 348: 1256-1266 (2003)
Non-Patent Document 4: Thorax 59: 960-965 (2004)
Non-Patent Document 5: Am. J. Respir. Crit. Care Med. 165: 867-903 (2002)
Non-Patent Document 6: Intensive Care Med. 22: 387-394 (1996)
Non-Patent Document 7: Lancet 362: 1991-2001 (2003)
Non-Patent Document 8: N. Engl. J. Med. 352: 380-391 (2005)

### Disclosure of the Invention

### Technical Problem

Acute respiratory infection is broadly classified into acute infection caused by pathogens that are usually absent in the respiratory tract (non-colonizing pathogens) and acute infection caused by pathogens colonizing the respiratory tract or respiratory pathogens causing chronic respiratory infection. The pathogens colonizing the respiratory tract or the respiratory pathogens causing chronic respiratory infection are confirmed to be also present in the respiratory tracts of healthy individuals, while they maintain the state of colonization or persistent infection. However, they shift to acute infection with a factor (e.g., the poor health of the carrier) as a trigger, causing strong symptoms (fever, pneumonia, septicemia, etc.). Thus, even if a pathogen likely to cause acute respiratory infection is detected by PCR or the like, whether this pathogen is a respiratory pathogen contributing to acute infection or is a colonizing pathogen or a respiratory pathogen causing chronic respiratory infection, which does not participate in acute infection, cannot be distinguished (Non-Patent Document 3). In fact, test using PCR detects the same pathogens both from the sputa or respiratory secretions of healthy individuals and from the sputa or respiratory secretions of pneumonia patients.

The present invention has been achieved in consideration of the problems of the conventional techniques, and an object of the present invention is to provide a method of distinguishing a respiratory pathogen causing acute respiratory infection.

### Solution to Problem

The present invention provides a method of distinguishing a respiratory pathogen causing acute respiratory infection, wherein
a pathogen to be distinguished includes a pathogen colonizing the respiratory tract, the method comprising:
measuring the relative value of the amount of the pathogen in a sample containing the respiratory secretions of a subject, to the amount of the subject's cells in the sample; and
distinguishing whether or not the pathogen is the respiratory pathogen, based on the relative value.

The prospective cohort study conducted in Thorax 59: 960-965 (2004) (Non-Patent Document 4) has showed that use of an inappropriate antibiotic in the initial treatment of community-acquired pneumonia requiring hospital treatment results in 11 times higher mortality than use of an appropriate antibiotic.

Am. J. Respir. Crit. Care Med. 165: 867-903 (2002) (Non-Patent Document 5) has reported that use of an appropriate antibiotic in the initial treatment of ventilator-associated pneumonia of hospital-acquired pneumonia differs with statistical significance in mortality from use of an inappropriate antibiotic.

Intensive Care Med. 22: 387-394 (1996) (Non-Patent Document 6) has reported that not all of the antibiotics used in the initial treatment of pneumonia requiring ICU treatment are appropriate, and examples of pathogens misidentified at high rates described therein include pathogens colonizing the respiratory tract, such as microbes of the genera *Pseudomonas*, *Staphylococcus*, and *Acinetobacter.* Specifically, the misidentification of the inflammatory microbe at the stage of initial treatment highly possibly hinders appropriate treatment from being accomplished, also increasing mortality.

According to the method of distinguishing a respiratory pathogen causing acute respiratory infection, of the present invention, whether the detected pathogen is the respiratory pathogen or a colonizing pathogen can be distinguished with higher sensitivity and higher specificity by distinguishing a pathogen colonizing the respiratory tract whose inflammatory state/colonizing state has been difficult to distinguish so far, based on comparison with the number of a subject's cells in the sample.

The present invention provides a method of determining a distinguishment reference value for distinguishing a respiratory pathogen causing acute respiratory infection, wherein
a pathogen to be distinguished is a pathogen colonizing the respiratory tract, the method comprising:
a) measuring a gene copy number derived from a subject's cells, in DNA prepared from a sample containing the respiratory secretions of the subject;
b) measuring a pathogen-derived gene copy number in the DNA;
c) calculating the relative number of the pathogen-derived gene copy number to the gene copy number derived from the subject's cells;
d) performing the a) to c) on a plurality of subjects to obtain the relative number for each of the subjects; and
e) comparing the relative number of each of the subjects with clinical diagnosis results of each of the subjects to thereby determine, as a distinguishment reference value:
   i) the lower limit of the relative number leading to respiratory pathogen positive in clinical diagnosis; and/or
   ii) the upper limit of the relative number leading to respiratory pathogen negative in clinical diagnosis.

The pathogen colonizing the respiratory tract is preferably one or more pathogens selected from the group consisting of microbes of the genera *Streptococcus*, *Haemophilus*, *Moraxella*, *Pseudomonas*, *Klebsiella*, *Stenotrophomonas*, *Acinetobacter*, and *Staphylococcus.* Moreover, the gene derived from the pathogen colonizing the respiratory tract is preferably a gene specific for the pathogen and is preferably a *Streptococcus pneumoniae* pneumolysin gene, a *Streptococcus pneumoniae* lytA gene, a *Haemophilus influenzae* 16S rRNA gene, a *Moraxella catarrhalis* copB gene, a *Pseudomonas* 16S rRNA gene, a *Klebsiella pneumoniae* gapA gene, a *Stenotrophomonas maltophilia* 23S rRNA gene, a *Staphylococcus aureus* femB gene, or the like.

According to the method of the present invention, a distinguishment reference value for distinguishing whether the pathogen colonizing the respiratory tract, detected in acute respiratory infection, is the respiratory pathogen or a colonizing pathogen, can be determined.

The present invention provides a method of distinguishing a respiratory pathogen causing acute respiratory infection, wherein
a pathogen to be distinguished is a pathogen colonizing the respiratory tract, the method comprising:
a) measuring a gene copy number derived from a subject's cells, in DNA prepared from a sample containing the respiratory secretions of the subj ect;
b) measuring a pathogen-derived gene copy number in the DNA;
c) calculating the relative number of the pathogen-derived gene copy number to the gene copy number derived from the subject's cells; and
d) distinguishing whether or not the pathogen is the respiratory pathogen, based on the distinguishment reference value and the relative number.

According to the method of distinguishing a respiratory pathogen causing acute respiratory infection, of the present invention, whether the detected pathogen is a respiratory pathogen or a colonizing pathogen can be distinguished with higher sensitivity and higher specificity by distinguishing a pathogen colonizing the respiratory tract whose inflammatory state/colonizing state has been difficult to distinguish so far, based on the distinguishment reference value serving as an objective index.

Moreover, for the method of distinguishing a respiratory pathogen causing acute respiratory infection, it is preferable to measure the gene copy number by a gene amplification method such as PCR. As a result, the respiratory pathogen can be distinguished rapidly. For example, results of distinguishing the respiratory pathogen are obtained within 4 hours from sample submission while a plurality of genes are tested. Thus, the respiratory pathogen can be distinguished appropriately at the early stage of initial treatment, and appropriate initial treatment can be performed.

Furthermore, since the number of subject-derived cells contained in the sample of respiratory secretions is also measured, it can be confirmed that sample collection, DNA preparation, PCR reaction, or the like are appropriately performed. Thus, incorrect determination (false negative) can be reduced in which results supposed to be positive are determined as negative due to poor DNA purification (e.g., mishandling of a purification kit), measurement errors of an operator (e.g., operator's forgetting about putting the sample into a measurement instrument), or the like. When the distinguishment results are negative, the involvement of the pathogen in the infection can be denied.

Furthermore, the method of distinguishing a respiratory pathogen causing acute respiratory infection, of the present invention, wherein
an additional pathogen to be distinguished is a pathogen that is usually absent in the respiratory tract, further comprises:
a) measuring a pathogen-derived gene copy number in DNA prepared from a sample containing the respiratory secretions of a subject; and
b) performing distinguishment such that:
   i) when the pathogen-derived gene is not detected, the pathogen does not participate in the infection; and
   ii) when the pathogen-derived gene is detected, the pathogen is the respiratory pathogen.

Examples of the pathogen that is usually absent in the respiratory tract include microbes of the genera *Mycoplasma, Legionella, Chlamydophila, Mycobacterium, Coxiella, Nocardia, Pneumocystis,* and *Aspergillus.* The gene derived from the pathogen that is usually absent in the respiratory tract is preferably a *Mycoplasma pneumoniae* 16S rRNA gene, a *Legionella pneumophila* mip gene, a *Legionella* 16S rRNA gene, a *Chlamydophila pneumoniae* 53-kD antigen gene, a *Chlamydophila psittaci* ompA gene, a *Mycobacterium tuberculosis* MPB64 gene, a *Mycobacterium intracellulare* ITS 16-23S rRNA gene, a *Mycobacterium avium* 16S rRNA gene, a *Mycobacterium avium* ITS 16-23S rRNA gene, a *Mycobacterium kansasii* dnaJ gene, a *Nocardia* 16S rRNA gene, a *Pneumocystis jiroveci* 5S rRNA gene, or the like.

The conventional diagnosis of a respiratory pathogen causing acute respiratory infection has various problems. First, Gram staining, a rapid diagnosis method most frequently used, is useful for some bacterial infections with a large bacterial content, whereas its clinical usefulness is lost for infections with a small bacterial content or with pathogens other than bacteria. Moreover, even when a bacterium is detected, this bacterium may be a colonizing pathogen or a respiratory pathogen causing chronic respiratory infection; thus it can often not be concluded that the bacterium is the respiratory pathogen causing acute respiratory infection. Urinary antigen study is available for *Streptococcus pneumoniae* and *Legionella* bacteria but cannot detect bacteria other than these two kinds. Moreover, since any urinary antigen test produces the results of positive over a long period after infection, it is sometimes difficult to diagnose infection at the time of the test. A pathogen that can be diagnosed by PCR is *Mycobacterium tuberculosis.* Since this diagnosis by PCR is useful as a confirmatory test for tuberculosis suspected in diagnostic imaging but cannot test other bacteria, this approach is unsuitable for the general diagnosis of a respiratory pathogen causing acute respiratory infection. Culture test that has previously been practiced has such problems that: it requires time for making diagnosis; whether the separated/isolated pathogen is the respiratory pathogen or a colonizing pathogen cannot be distinguished; and culture often fails due to the preemptive administration of antibiotics, if any. Moreover, this approach has such problems that pathogens are cultured only in media suitable therefor, and the respiratory pathogen cannot be distinguished unless the test is practiced using a large number of media combined.

According to the method of distinguishing a respiratory pathogen causing acute respiratory infection, of the present invention, a pathogen that is usually absent in the respiratory tract can be tested simultaneously with the pathogen colonizing the respiratory tract. Specifically, pathogens of acute respiratory infection can be detected comprehensively. Accordingly, analysis results can be compared easily among different pathogens. Since all pathogens detected with high frequency in community-acquired pneumonia and hospital-acquired pneumonia are included therein, the distinguishment method of the present invention is very clinically useful from the viewpoint that it can comprehensively distinguish the main respiratory pathogen.

In addition, in the method of distinguishing a respiratory pathogen causing acute respiratory infection, of the present invention, the pathogen-derived gene further includes a gene associated with the acquisition of drug resistance of a pathogen (drug resistance gene). The drug resistance gene is preferably a mecA gene or a metallo-β-lactamase gene, or the like.

As a result, the drug resistance gene can also be searched comprehensively, simultaneously with the pathogen to rapidly obtain comprehensive test results. Accordingly, whether the drug-resistant pathogen is a colonizing pathogen or the respiratory pathogen can be distinguished. Thus, information useful for antibiotic selection can be provided. Furthermore, since the presence of a drug-resistant pathogen having a drug resistance gene can be detected and, for example, the presence or absence of drug-resistant microbe carry of a hospitalized patient can also be examined, the distinguishment method of the present invention is also excellent from a preventive standpoint such as a measure against hospital-acquired infection.

Moreover, the subject is preferably a mammal, particularly preferably, a human. When the subject is a human, the gene derived from the subject's cells is preferably a human-specific gene derived from the human cells and is preferably, for example, a human SFTPC (surfactant, pulmonary-associated protein C) gene, a β-globin gene, or a β-actin gene.

The present invention provides for the use of a kit for performing the methods of the invention. The kit comprises: a primer set for a gene derived from one or more pathogens colonizing the respiratory tract, selected from the group consisting of microbes of the genera *Streptococcus*, *Haemophilus*, *Moraxella*, *Pseudomonas*, *Klebsiella*, *Stenotrophomonas*, *Acinetobacter*, and Staphylococcus; and a manual.

It is preferable that the kit should further comprise: a primer set for a gene derived from one or more pathogens that are usually absent in the respiratory tract, selected from the group consisting of microbes of the genera *Mycoplasma, Legionella, Chlamydophila*, *Mycobacterium*, *Coxiella*, *Nocardia*, *Pneumocystis*, and *Aspergillus*; and a manual. It is also preferable that the kit should further comprise: a primer set for one or more drug resistance genes selected from the group consisting of a mecA gene and a metallo-β-lactamase gene; and a manual.

According to the kit for distinguishing a respiratory pathogen causing acute respiratory infection, the respiratory pathogen causing acute respiratory infection can be distinguished from a colonizing pathogen with higher sensitivity and higher specificity than the existing tests.

The present invention provides for the use of an apparatus for performing the methods of the invention. The apparatus, wherein a pathogen to be distinguished is a pathogen colonizing the respiratory tract, comprises the following means:
a) DNA preparation means for preparing DNA from a sample containing the respiratory secretions of a subject;
b) DNA amplification means for specifically amplifying DNA derived from the subject's cells and pathogen-derived DNA, in the DNA;
c) DNA detection means for detecting the DNA derived from the subject's cells and the pathogen-derived DNA, amplified by the DNA amplification means;
d) calculation means for calculating the relative number of a copy number of the pathogen-derived DNA to a copy number of the DNA derived from the subject's cells, based on a signal detected by the DNA detection means; and
e) distinguishment means for distinguishing whether or not the pathogen is the respiratory pathogen, based on the distinguishment reference value and the relative number.

Moreover, the apparatus, wherein an additional pathogen to be distinguished is a pathogen that is usually absent in the respiratory tract, further comprises the following means:
a) DNA preparation means for preparing DNA from a sample containing the respiratory secretions of a subject;
b) DNA amplification means for specifically amplifying a pathogen-derived DNA in the DNA;
c) DNA detection means for detecting the pathogen-derived DNA amplified by the DNA amplification means;
d) calculation means for calculating a copy number of the pathogen-derived DNA based on a signal detected by the DNA detection means; and
e) distinguishment means for performing distinguishment such that:
   i) when the pathogen-derived DNA is not detected, the pathogen does not participate in the infection; and
   ii) when the pathogen-derived DNA is detected, the pathogen is the respiratory pathogen.

For the apparatus, it is preferable that the pathogen-derived DNA should further include a drug resistance gene.

According to the apparatus, a large number of samples and a large number of pathogens or drug resistance genes per sample can be tested in a short time to distinguish the respiratory pathogen causing acute respiratory infection.

Furthermore, the method of distinguishing a respiratory pathogen causing acute respiratory infection and the kit and apparatus for distinguishing a respiratory pathogen causing acute respiratory infection are excellent in flexibility and can thereby further enhance convenience, because viruses participating in respiratory infection or fungi which have been difficult to rapidly diagnose so far can also be added as pathogens to be tested.

The existing tests require high cost (26,000 yen or higher) only for searching these pathogens. In the present invention, low cost (4,600 yen) can be achieved even if comprehensive search of 21 kinds of target genes per sample is performed. The present invention achieves reduction in medical cost greatly exceeding the test cost and is also very promising from the viewpoint of market.

The existing tests require at least 3 to 14 days for confirming the diagnosis, even in culture test as a main detection method, and require at last 14 days when paired serum is used. By contrast, the present invention can achieve sample treatment through comprehensive search of a plurality of target genes and return of the test results in 4 hours. Thus, the present invention is also excellent from the viewpoint of rapidity.

Since Gram staining used in the existing rapid diagnosis requires technical skills and ability for image reading thereof, results may differ depending on the test operator. However, PCR does not need technical skills and can also be compiled into a manual. Moreover, even different test operators can get highly precise results. Thus, the present invention is excellent in terms of high objectivity.

### Brief Description of Drawings

Fig. 1 is a scheme schematically showing the principles of respiratory infection;
Fig. 2 is a block diagram showing the configuration of an apparatus for distinguishing a respiratory pathogen causing acute respiratory infection according to one example of the present invention;
Fig. 3 is a graph showing the relationship between a PCR cycle number and fluorescence intensity in the detection of target genes using 2 actual cases of pneumonia, and a photograph of Gram-stained sputa;
Fig. 4 is a graph plotting ΔCycle vs. clinical diagnosis results in 44 cases (Fig. 4A) and 100 cases (Fig. 4B) from which *Streptococcus pneumoniae* was detected;
Fig. 5 is a graph plotting ΔCycle vs. clinical diagnosis results in 26 cases (Fig. 5A) and 29 cases (Fig. 5B) from which *Haemophilus influenzae* was detected;
Fig. 6 is a graph plotting ΔCycle vs. clinical diagnosis results in 37 cases (Fig. 6A) and 48 cases (Fig. 6B) from which *Pseudomonas aeruginosa* was detected;
Fig. 7 is a graph plotting ΔCycle vs. clinical diagnosis results in 17 cases (Fig. 7A) and 20 cases (Fig. 7B) from which *Moraxella catarrhalis* was detected;
Fig. 8 is a graph plotting ΔCycle vs. clinical diagnosis results in 34 cases from which methicillin-resistant *Staphylococcus aureus* was detected;
Fig. 9 is a graph showing the breakdown of 158, out of 300 cases, from which a respiratory pathogen was identified. The graph shows a pathogen name, the number of cases, and the rate of cases (%);
Fig. 10 is a graph showing the breakdown of distinguishment results of 223 cases. The graph shows a pathogen name and the rate of cases (%); and
Fig. 11 is a graph showing the breakdown of distinguishment results of 174 cases enrolled in multicenter prospective trial. The graph shows a pathogen name and the rate of cases (%).

### Best Modes for Carrying Out the Invention

Hereinafter, the preferred embodiments of the present invention will be described in detail.

A method of distinguishing a respiratory pathogen causing acute respiratory infection, of the present embodiment, wherein a pathogen to be distinguished includes a pathogen colonizing the respiratory tract, comprises: measuring the relative value of the amount of the pathogen in a sample containing the respiratory secretions of a subject, to the amount of the subject's cells in the sample; and distinguishing whether or not the pathogen is the respiratory pathogen, based on the relative value.

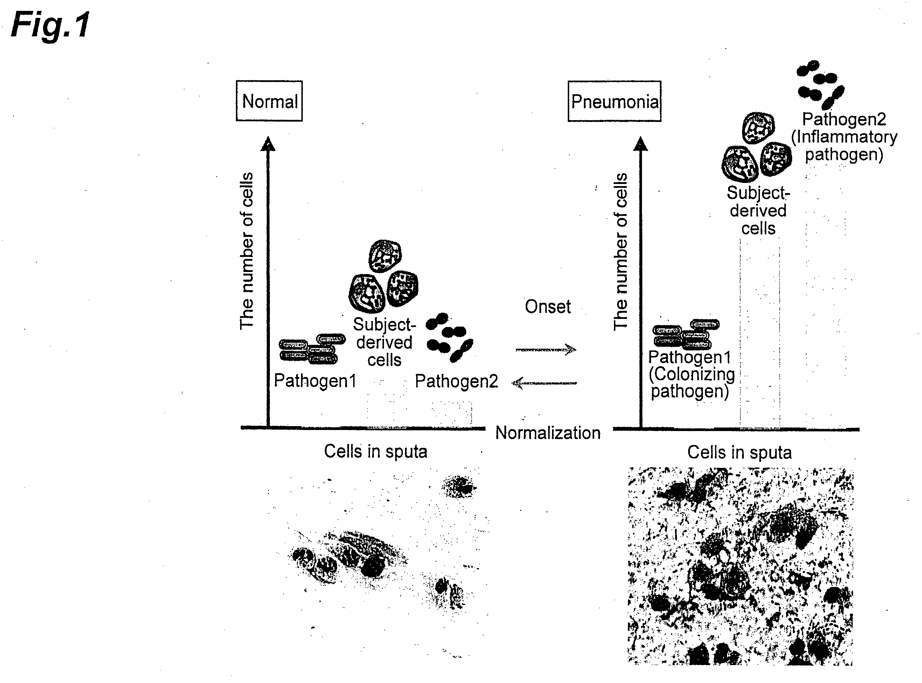

First, the principles of the method of distinguishing a respiratory pathogen causing acute respiratory infection, of the present invention, will be described with reference to Fig. 1. The left graph and photograph of Fig. 1 show the presence of pathogens (as an example, a pathogen 1 and a pathogen 2) present in the sputa or respiratory secretions of a normal subject (healthy individual), and subject-derived cells (human cells). The pathogen 1 and the pathogen 2 are pathogens colonizing the respiratory tract, and these pathogens do not cause inflammation while the subject as a carrier does not develop respiratory symptoms. Here, assume that acute respiratory infection was caused by the pathogen 2. The rates of the bacterium and the subject-derived cells in the sputa or respiratory secretions of the subject affected by acute respiratory infection change as shown in the right diagram of Fig. 1. Due to the intense inflammation in the respiratory tract, the subject develops respiratory symptoms (dyspnea, cough, and purulent sputa). The pathogen 2 as a respiratory pathogen also increases. No apparent change is seen in the pathogen 1, which does not participate in the pneumonia. This demonstrates that the pathogen 1 is a colonizing pathogen.

Here, when the pathogen 1 and the pathogen 2 are detected by a method known in the art such as PCR, both of them are detected. It is diffcult to estimate the abundance (abundance ratio) of the pathogen of interest according to the difference in DNA purification efficiency, target gene amplification/detection efficiency, or the like between the pathogens. Furthermore, the test on the subject-derived sputa or respiratory secretions using PCR known in the art can measure merely the presence or absence of the pathogen and the amount of the pathogen per unit volume. The sputa or respiratory secretions are often contaminated with a large amount of pathogens colonizing saliva or the upper respiratory tract, and it is thus difficult to determine the respiratory pathogen causing acute respiratory infection, from information about the presence or absence of the pathogen and the amount of the pathogen per unit volume. In other words, the sputa/respiratory secretions are too largely diverse to be collectively handled as the same kinds of samples. Accordingly, even if the test is conducted by a known approach using the fixed amount of samples or DNAs used in detection, the detection results are difficult to normalize and, in most cases, are not easy to interpret.

The present invention focused on subject-derived cells commonly present in respiratory secretions (respiratory epithelial cells or inflammatory cells such as leukocytes). As shown in the right diagram of Fig. 1, the subject-derived cells increase to calm the activity of pathogens at the onset of acute respiratory infection. On the other hand, presumably, the amount of colonizing pathogens unrelated to inflammation hardly changes. Thus, it was hypothesized that the relative number of the abundance of the pathogen can be estimated with the subject-derived cells as an internal control to thereby distinguish the respiratory pathogen from a colonizing pathogen. This hypothesis was proved in Examples described later, and the present invention has been completed.

Hereinafter, main respiratory pathogens causing acute respiratory infection classified according to where they occur will be described. Community-acquired pneumonia (CAP) refers to pneumonia that affects ordinary people or the like in the society. Table 1 lists main respiratory pathogens causing community-acquired pneumonia (see Non-Patent Documents 3 and 7). The community-acquired pneumonia can be caused by a large number of respiratory pathogens including pathogens colonizing the respiratory tract and pathogens that are usually absent in the respiratory tract. Therefore, the disease requires comprehensively testing a large number of pathogens to determine the main responsible pathogen causing acute respiratory infection. For this purpose, two stages are necessary. The first stage is a process of comprehensively detecting possible pathogens by a highly sensitive approach. The second stage is a process of distinguishing, of the detected pathogens, the respiratory pathogens from colonizing pathogens. The distinguishment method of the present invention achieves comprehensive detection and distinguishment of pathogens.

**[Table 1]**

| Community-acquired pneumonia | |
|---|---|
| Pathogen | Classification |
| *Streptococcus pneumonia* | CO |
| *Mycoplasma pneumonlae* | NCO |
| *Chlamydophila pneumoniae* | NCO |
| *Haemophilus influenzae* | CO |
| *Staphylococcus aureus* | CO |
| *Moraxella catarrhalis* | CO |
| Legionella spp. | NCO |
| *Enterobacteriaceae* | CO |
| *Pseudomonas* spp. | CO |
| Anaerobes | CO |
| Virus | |
| *Pneumocystis* spp. | |
| *Mycobacterium tuberculosis* | NCO |
| *Chlamydophila psittaci* | NCO |
| *Coxiella bumetii* | NCO |

| | |
|---|---|
| CO: pathogen colonizing the respiratory tract NCO: pathogen that is usually absent in the respiratory tract | |

Ventilator-associated pneumonia (VAP) is one class of hospital-acquired pneumonia and refers to pneumonia in ventilator-dependent patients. Table 2 lists main respiratory pathogens causing ventilator-associated pneumonia (see Non-Patent Document 5). As is evident from Table 2, most of the main respiratory pathogens are pathogens colonizing the respiratory tract or respiratory pathogens causing chronic respiratory infection. Thus, it is highly necessary to distinguish whether the detected pathogens are the respiratory pathogens or colonizing pathogens. The present invention allows distinguishment of pathogens colonizing the respiratory tract, which have never been distinguished so far.

**[Table 2]**

| Ventilator-associated pneumonia | | | |
|---|---|---|---|
| Pathogen | | Frequency (%) | Classification |
| *Pseudomonas aeruginosa* | | 24.4 % | CO |
| *Acinetobacter* spp. | | 7.9 % | CO |
| *Stenotrophomonas maltophilia* | | 1.7 % | CO |
| *Enterobacteriaceae* | *Klebsiella* spp. | 2.2 % | 14.1 % CO |
| | *E*. *coil* | 3.4 % | CO |
| | *Enterabacter* spp. | 2.7 % | CO |
| | others | 5.8 % | CO |
| *Haemophilus* spp. | | 9.8 % | CO |
| *Staphylococcus aureus* | *MSSA* | 10.0 % | 20.4 % CO |
| | *MRSA* | 11.4 % | CO |
| *Streptococcus* spp. | | 8.0 % | CO |
| *Streptococcus pneumoniae* | | 4.1 % | CO |
| Cagulase-negative staphylococci | | 1.4 % | CO |
| *Neisseria* spp. | | 2.6 % | CO |
| Anaerobes | | 0.9 % | CO |
| Fungi | | 0.9 % | |
| Others | | 3.8 % | |

| | | | |
|---|---|---|---|
| CO: pathogen colonizing the respiratory tract | | | |

Next, a method of determining a distinguishment reference value for distinguishing a respiratory pathogen causing acute respiratory infection and a method of distinguishing a respiratory pathogen causing acute respiratory infection, based on the distinguishment reference value, according to embodiments of the present invention, will be described.

### (Method of determining a distinguishment reference value for distinguishing a respiratory pathogen causing acute respiratory infection)

The method of determining a distinguishment reference value for distinguishing a respiratory pathogen causing acute respiratory infection is a method of determining a distinguishment reference value for distinguishing a pathogen colonizing the respiratory tract and comprises the following:
preparing DNA from a sample containing the respiratory secretions of a subject;
   a) measuring a gene copy number derived from the subject's cells, in the DNA;
   b) measuring a pathogen-derived gene copy number in the DNA;
   c) calculating the relative number of the pathogen-derived gene copy number to the gene copy number derived from the subject's cells;
   d) performing the a) to c) on a plurality of subjects to obtain the relative number for each of the subjects; and
   e) comparing the relative number of each of the subjects with clinical diagnosis results of each of the subjects to thereby determine, as a distinguishment reference value:
      i) the lower limit of the relative number leading to respiratory pathogen positive in clinical diagnosis; and/or
      ii) the upper limit of the relative number leading to respiratory pathogen negative in clinical diagnosis.

Subsequently, each step will be described.

The pathogen in the present embodiment is a pathogen colonizing the respiratory tract. The "pathogen colonizing the respiratory tract" is a pathogen capable of colonizing the respiratory tract (also referred to as a colonizing pathogen, an indigenous pathogen, a commensal pathogen, or a commensal organism) and is also present in healthy individuals free from infection. In the normal state, these colonizing pathogens do not cause infection while the carrier (subject) does not develop respiratory symptoms. However, they can shift to infection with a factor (e.g., the poor health of the subject) as a trigger to become causative pathogens (respiratory pathogens) of chronic/acute respiratory infection. In the present invention, the "pathogen colonizing the respiratory tract" refers to the kind of a pathogen capable of colonizing the respiratory tract. Moreover, simply the "colonizing pathogen" refers to a pathogen colonizing the respiratory tract, in a state uninvolved in the respiratory infection.

Examples of the pathogen colonizing the respiratory tract include: microbes of the genus *Streptococcus,* such as *Streptococcus pneumoniae;* microbes of the genus *Haemophilus,* such as *Haemophilus influenzae;* microbes of the genus *Moraxella,* such as *Moraxella catarrhalis;* microbes of the genus *Pseudomonas,* such as *Pseudomonas aeruginosa;* microbes of the genus *Klebsiella,* such as *Klebsiella pneumoniae;* microbes of the genus *Stenotrophomonas,* such as *Stenotrophomonas maltophilia*; microbes of the genus *Acinetobacter,* such as *Acinetobacter baumannii*; and microbes of the genus *Staphylococcus,* such as *Staphylococcus aureus.*

The subject is preferably a mammal, particularly, a human. Moreover, the subject is a subject having acute respiratory infection such as acute pneumonia or a subject suspected of having it. Any sample containing the respiratory secretions is available, and specifically, the sample is preferably sputa such as expectorated sputa, aspirated sputa, induced sputa, or intratracheally collected sputa. Moreover, the sample is preferably sputa immediately after being expectorated or sputa cryopreserved at -20°C.

The DNA refers to subject-derived DNA and/or pathogen-derived DNA, i.e., genomic DNA, mitochondrial DNA, or complementary DNA converted from genomic RNA to DNA with reverse transcriptase. The DNA used in detection may be prepared such that gene DNA present in the DNA can be detected. The DNA extraction/purification method is known in the art, and the DNA can be prepared using a method generally known by those skilled in the art described in, for example, Sambrook et al. ((2001) Molecular Cloning: a Laboratory Manual, 3rd ed., Cold Spring Harbor Laboratory Press) or a commercially available DNA purification kit.

The phrase "measuring a gene copy number" refers to measuring the number of copies of the targeted gene sequence in the subject-derived DNA and/or the pathogen-derived DNA. Moreover, it refers to measuring the relative copy number of the target DNA that can be compared in the sample. The measurement of the relative DNA copy number may be performed using a measurement system generally known by those skilled in the art, and specific examples thereof include gene amplification detection methods such as PCR (real-time PCR). Examples of PCR quantification methods include agarose gel electrophoresis, intercalator method (SYBR Green method), and fluorescence probe method. Among others, real-time PCR using the fluorescence probe method is preferable. The relative DNA copy number can be represented by a Cycle threshold (Ct value) in real-time PCR using the intercalator or fluorescence probe method.

A primer set used in PCR recognizes a specific region of the gene to specifically recognize the subject's or pathogen's gene, and the specific nucleotide sequences of the primers can be determined appropriately.

A fluorescence probe used in the fluorescence probe method can be selected from TaqMan probes, cycling probes, Molecular Beacon, and so on. The fluorescence probe recognizes a specific region of the gene to specifically recognize the subject's or pathogen's gene, together with the primer set, and the specific nucleotide sequence of the probe can be determined appropriately. The fluorescence probe is modified at its 5' and 3' ends with a fluorescent material and a quencher material, respectively, and fluorescence is detected which is emitted due to cancellation of quenching of the quencher material as the PCR reaction proceeds. As a result, the relative copy number of the target gene sequence in the DNA can be measured. The combination of the fluorescent material and the quencher material needs only to be a combination of materials that can respectively be identified, and the combination of such fluorescently labeling materials that can be used is a combination of those generally known by those skilled in the art and is preferably a combination of FAM (registered trademark) and TAMRA (registered trademark), TET (registered trademark) and BHQ-1 (registered trademark), or the like. Moreover, use of a system of simultaneously exciting and detecting fluorescences of different wavelengths (e.g., Smart Cycler II System, TAKARA BIO INC.) can simultaneously detect a plurality of target sequences amplified through one multiplex PCR reaction. PCR reaction through which one target nucleotide sequence is amplified with a pair of PCR primers is referred to as single PCR, whereas PCR reaction through which a plurality of target nucleotide sequences are simultaneously amplified with a plurality of pairs of PCR primers is referred to as multiplex PCR.

The phrase "calculating the relative number" refers to calculating the relative copy number, specifically, calculating the relative number of the relative copy number of the pathogen-derived gene sequence, to the relative copy number of the gene sequence derived from the subject's cells. As a result, the relative number of the pathogen cells can be estimated with the number of subject-derived cells as an internal control and can be compared among different samples. In other words, the a) to c) are pathogen detection steps, by which the pathogen can be normalized and detected. For example, the relative number of pathogen cells can be represented by a value (ΔCycle) determined by subtracting a Ct value obtained in the pathogen-derived gene from a Ct value obtained in the gene derived from the subject's cells.

The "clinical diagnosis results" are clinical diagnosis results of acute respiratory infection available in the art and refer to observations obtained in clinical diagnosis in such a way that the respiratory pathogen is identified, the respiratory pathogen is suspected, or a certain pathogen is determined not to be the respiratory pathogen, by a test method known in the art such as Gram staining of sputum smear, sputum culture method, urinary antigen test, or paired serum method.

Moreover, the "respiratory pathogen positive in clinical diagnosis" means that the respiratory pathogen has been identified, or the respiratory pathogen has been suspected, in the diagnosis. The "respiratory pathogen negative in clinical diagnosis" means that a certain pathogen has been determined not to be the respiratory pathogen, in the diagnosis.

To determine the distinguishment reference value, first, the relative number (e.g., ΔCycle) obtained in each of the subjects and clinical diagnosis results showing respiratory pathogen positive/negative, obtained in each of the subjects, are plotted together, and the correlation therebetween is statistically analyzed. Next, the relative number leading to the respiratory pathogen/colonizing pathogen (non-respiratory pathogen) threshold of the pathogen of interest, i.e., the lower limit of the relative number leading to significant respiratory pathogen positive in clinical diagnosis and/or the upper limit of the relative number leading to significant respiratory pathogen negative in clinical diagnosis, is determined, and this is used as a distinguishment reference value. Such a statistical analysis method is generally known by those skilled in the art. In this way, for the particular target gene of a certain pathogen, a distinguishment reference value unique thereto can be determined. Moreover, the number of samples is further added, and the relative numbers and clinical diagnosis results of these samples can be added to the plot to thereby evaluate the reliability of the distinguishment reference value and determine a more highly reliable distinguishment reference value.

### (Method of distinguishing a respiratory pathogen causing acute respiratory infection)

The method of distinguishing a respiratory pathogen causing acute respiratory infection, of the present embodiment, is broadly classified into the following three methods:
(1) a distinguishment method, wherein a pathogen to be distinguished is a pathogen colonizing the respiratory tract,
(2) a distinguishment method, wherein an additional pathogen to be distinguished is a pathogen that is usually absent in the respiratory tract, and
(3) a distinguishment method, further comprising measuring a drug resistance gene copy number and evaluating the drug resistance of a pathogen.
Hereinafter, each method will be described.

### (1) Distinguishing method, wherein a pathogen to be distinguished is a pathogen colonizing the respiratory tract

The method of distinguishing a respiratory pathogen causing acute respiratory infection, wherein a pathogen to be distinguished is a pathogen colonizing the respiratory tract, comprises the following: preparing DNA from a sample containing the respiratory secretions of a subject;
a) measuring a gene copy number derived from the subject's cells, in the DNA;
b) measuring a pathogen-derived gene copy number in the DNA;
c) calculating the relative number of the pathogen-derived gene copy number to the gene copy number derived from the subject's cells; and
d) distinguishing whether or not the pathogen is the respiratory pathogen, based on the distinguishment reference value and the relative number.

The DNA preparation to the c) may be performed in the same way as in the method of determining a distinguishment reference value, and the overlapping description will be omitted.

Pathogens colonizing the respiratory tract shift to acute infection, i.e., an inflammatory state, with a factor (e.g., the poor health of the carrier) as a trigger through the processes of adherence to the respiratory tract, proliferation, colonization, and infection. Thus, for the pathogens colonizing the respiratory tract, there exist 3 kinds of states (distinguishment results): negative, colonizing pathogens, and respiratory pathogens. Likewise, for opportunistic pathogens, which participate in infection through similar processes, there exist 3 kinds of states (distinguishment results): negative, colonizing pathogens (state of inapparent infection), and respiratory pathogens.

In the distinguishment d) in the method (1), the obtained relative number can be compared with the distinguishment reference value to thereby distinguish which of these 3 kinds of states the pathogen belongs to. Specifically, the distinguishment is performed such that: i) when the gene derived from the pathogen colonizing the respiratory tract is not detected, the pathogen is not colonizing the respiratory tract (negative); ii) when the gene derived from the pathogen colonizing the respiratory tract is detected, albeit with the relative number below the distinguishment reference value, the pathogen is not the respiratory pathogen but a colonizing pathogen; and iii) when the relative number is equal to or above the distinguishment reference value, the pathogen is the respiratory pathogen. In this context, the phrase "not detected" or below or equal to the detection limit means that, for example, when the relative gene copy number is represented by a Ct value, signal intensity (fluorescence intensity, etc.) derived from the amplification product of the gene does not reach signal intensity that defines the Ct value. When the gene derived from the subject's cells used as an internal control is not detected, the relative number is not obtained: thus this case is not a subject to be distinguished. In this case, sample collection, DNA preparation, PCR reaction, or the like is suspected of being inappropriately performed. Thus, false-negative, which has been determined to be negative in the conventional test, can be reduced.

### (2) Distinguishment method, wherein an additional pathogen to be distinguished is a pathogen that is usually absent in the respiratory tract

Next, the method of distinguishing a respiratory pathogen causing acute respiratory infection, wherein in addition to the pathogen colonizing the respiratory tract, a pathogen that is usually absent in the respiratory tract is used as an additional pathogen to be distinguished, will be described. The distinguishment method of the present embodiment comprises the steps of the distinguishment method (1) and further comprises the following:
preparing DNA from a sample containing the respiratory secretions of a subj ect;
   a) measuring a pathogen-derived gene copy number in the DNA prepared from the sample containing the respiratory secretions of a subject; and
   b) performing distinguishment such that:
      i) when the pathogen-derived gene is not detected, the pathogen does not participate in the infection; and
      ii) when the pathogen-derived gene is detected, the pathogen is the respiratory pathogen.

Examples of the pathogen that is usually absent in the respiratory tract (noncommensal organism) include: microbes of the genus *Mycoplasma,* such as *Mycoplasma pneumoniae*; microbes of the genus *Legionella,* such as *Legionella pneumophila;* microbes of the genus *Chlamydophila,* such as *Chlamydophila pneumoniae* and *Chlamydophila psittaci;* microbes of the genus *Mycobacterium,* such as *Mycobacterium tuberculosis, Mycobacterium intracellulare, Mycobacterium avium,* and *Mycobacterium kansasii;* microbes of the genus *Coxiella,* such as *Coxiella burnetii;* and opportunistic infectious pathogens, i.e., microbes of the genus *Nocardia,* such as *Nocardia asteroides,* microbes of the genus *Pneumocystis,* such as *Pneumocystis jiroveci,* and microbes (fungi) of the genus *Aspergillus,* such as *Aspergillus fumigatus.*

Opportunistic infection is infection that occurs due to pathogens that do not cause infection in healthy subjects (also referred to as attenuated microorganisms, nonpathogenic microorganisms, or opportunistic organisms). In a subject with acquired immunodeficiency syndrome (AIDS) or in an immune-compromised state caused by steroid, immunosuppressive agents, or the like, a low virulent pathogen whose proliferation is normally suppressed by his/her immunity may proliferate and, as a result, cause the disease. Pathogens generally participating in opportunistic infection are broadly classified into exogenous pathogens that exist in the environment and sometimes invade subjects and into endogenous pathogens that latently exist in humans, and the former and the latter are treated as an opportunistic pathogen and as a pathogen colonizing the respiratory tract, respectively, in the present invention.

Since the pathogen that is usually absent in the respiratory tract does usually not reside in the respiratory tract, there exist two kinds of distinguishment results: negative and respiratory pathogens. In the distinguishment b) in the method (2), which of these 2 kinds of states the pathogen belongs to can be distinguished based on the presence or absence of detection of a pathogen that is usually absent in the respiratory tract. Specifically, the distinguishment is performed such that: i) when the pathogen-derived gene is not detected, the pathogen does not participate in the infection (negative); and ii) when the pathogen-derived gene is detected, the pathogen is the respiratory pathogen.

In addition to the pathogens listed above, viruses, bacteria, fungi, and/or parasites, etc. that participate in respiratory infection may be detected additionally. Examples of the viruses include influenza virus, , parainfluenza virus, adenovirus, rhinovirus, RS virus, metapneumovirus, and cytomegalovirus (CMV). Examples of the bacteria include microbes of the genus *Acinetobacter,* such as *Acinetobacter baumannii,* and microbes of the genus *Coxiella,* such as *Coxiella burnetii.* Examples of the fungi include microbes of the genus *Aspergillus,* such as *Aspergillus fumigatus,* and microbes of the genus *Cryptococcus,* such as *Cryptococcus neoformans.* Examples of the parasites include lung flukes such as *Paragonimus westermani.*

### (3) Distinguishment method, further comprising measuring a drug resistance gene copy number and evaluating the drug resistance of a pathogen

The third distinguishment method comprises the distinguishment method (1) or (2) and further comprises measuring a drug resistance gene copy number and evaluating the drug resistance of a pathogen. In this distinguishment method, a gene associated with the acquisition of drug resistance of a pathogen (drug resistance gene) can be further added as a pathogen-derived gene and detected/ distinguished.

Examples of the gene conferring resistance to a drug such as an antibiotic (drug resistance gene) includes: methicillin resistance genes such as mecA gene; metallo-β-lactamase genes (IMP gene, VIM gene, GIM-1 gene, SPM-1 gene, etc.; see "The New β-lactamases" in Non-Patent Document 8) encoding an enzyme hydrolyzing most of antibiotics having a β-lactam ring, such as penicillin, cephem, and carbapenem; isoniazid (INH) resistance genes such as katG gene, inhA gene, and ahpO gene; rifampicin (RFP) resistance genes such as rpoB gene; ethambutol (EB) resistance genes such as embC gene, embB gene, and embA gene; streptomycin (SM) resistance genes such as rpsL gene and rrs gene; pyrazinamide (PZA) resistance genes such as PncA gene; and quinolone (e.g., fluoroquinolone) drug resistance genes such as gyrA gene and gyrB gene. Among them, the clinically important drug resistance genes mecA gene and metallo-β-lactamase gene are preferable.

Methicillin-resistant *Staphylococcus aureus* (MRSA) or multidrug-resistant *Pseudomonas aeruginosa* is known as a drug-resistant pathogen. Moreover, *Mycobacterium tuberculosis* resistant to drugs such as INH, RFP, EB, SM, PZA, and quinolones has been reported. Examples of the method for evaluating the drug resistance of a pathogen include a method wherein the relative number obtained from a pathogen-specific gene is compared with the relative number obtained from the pathogen-derived drug resistance gene to estimate the presence of the drug-resistant pathogen from the ratio of the relative copy number of the drug resistance gene to the relative copy number of the pathogen-specific gene. Specifically, when equivalent amounts of *Staphylococcus aureus* femB genes and methicillin resistance genes mecA are detected, the presence of methicillin-resistant *Staphylococcus aureus* is suggested. Alternatively, when equivalent amounts of metallo-β-lactamase genes and *Pseudomonas* 16S rRNA genes are detected, the presence of multidrug-resistant *Pseudomonas aeruginosa* is suggested.

Moreover, even if the relative copy number of the drug resistance gene is small, the risk of allowing a pathogen having the drug resistance gene to become a main respiratory pathogen in the future is presented. Moreover, the risk of allowing other pathogens to acquire the drug resistance gene and to lead to the emergence of, for example, methicillin-resistant *Staphylococcus aureus* or multidrug-resistant *Pseudomonas aeruginosa* in the future is presented. Therefore, the evaluation of the drug resistance of a pathogen offers useful information from the aspects of antibiotic selection in treatment and a measure against hospital-acquired infection.

### (Kit for distinguishing a respiratory pathogen causing acute respiratory infection)

A kit is for distinguishing a respiratory pathogen causing acute respiratory infection, by the method of distinguishing a respiratory pathogen causing acute respiratory infection and comprises: a primer set for a gene derived from one or more of the pathogens colonizing the respiratory tract; and a manual. Moreover, it is preferable that the kit should further comprise: a primer set for a gene derived from one or more of the pathogens that are usually absent in the respiratory tract; and a manual, and/or should further comprise: a primer set for one or more of the drug resistance genes; and a manual.

It is preferable that each primer set should be designed for a gene region specific for the targeted pathogen or should be designed for a gene region specific for the drug resistance gene. Each primer set may comprise a probe specific for the gene region to be amplified, and it is preferable to amplify and detect the pathogen-derived gene by fluorescence probe method. Specifically, primer sets listed in Table 3 can be used. The manual comprises instructions for using the method of distinguishing a respiratory pathogen causing acute respiratory infection and distinguishment reference values for individual pathogens and/or drug resistance genes or a method of determining the distinguishment reference values.

### (Apparatus for distinguishing a respiratory pathogen causing acute respiratory infection)

An apparatus for distinguishing a respiratory pathogen causing acute respiratory infection, of the present embodiment (hereinafter, referred to as a distinguishment apparatus), will be described. The distinguishment apparatus is broadly classified into the following three manners:
(1) a distinguishment apparatus, wherein a pathogen to be distinguished is a pathogen colonizing the respiratory tract;
(2) a distinguishment apparatus, wherein an additional pathogen to be distinguished is a pathogen that is usually absent in the respiratory tract; and
(3) a distinguishment apparatus for further measuring a drug resistance gene copy number and evaluating the drug resistance of a pathogen.

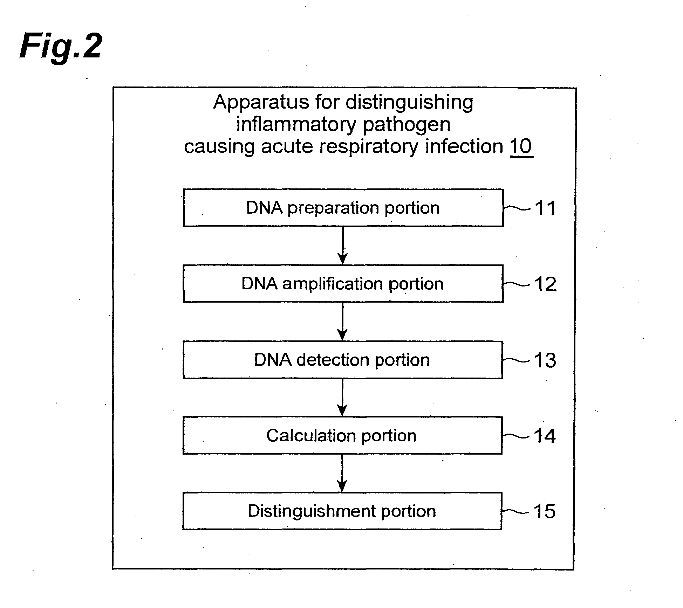

Fig. 2 is a block diagram of the apparatus. Hereinafter, the apparatus will be described in detail with reference to Fig. 2. In this diagram, a distinguishment apparatus 10 comprises a DNA preparation portion 11, a DNA amplification portion 12, a DNA detection portion 13, a calculation portion 14, and a distinguishment portion 15. The distinguishment apparatus 10 can automatically perform all the DNA preparation/amplification/detection/calculation/distinguishment procedures, and each procedure is performed in the DNA preparation portion 11, the DNA amplification portion 12, the DNA detection portion 13, the calculation portion 14, and the distinguishment portion 15, in this order, placed within the distinguishment apparatus 10. Thus, according to the distinguishment apparatus 10, a test operator can accomplish the distinguishment of the respiratory pathogen simply by loading a sample tube supplemented with a sample containing the respiratory secretions of a subject, to a sample tube holder in the DNA preparation portion 11 and then actuating the distinguishment apparatus 10.

Next, the operation of the distinguishment apparatus having such configuration will be described. Upon actuation of the distinguishment apparatus 10, the sample containing the respiratory secretions of a subject is introduced into the DNA preparation portion 11, by which DNA is then prepared from the sample. The DNA preparation portion 11 performs DNA preparation by treating the cell-containing sample with a surfactant and proteinase K added, followed by arbitrary DNA extraction means including porous membrane (e.g., silica gel membrane)-based DNA purification methods (e.g., QIAamp DNA Blood Kit (registered trademark), QIAGEN). The DNA amplification portion 12 amplifies DNA derived from the pathogen to be distinguished (DNA corresponding to a region specific for a pathogen gene) with the DNA prepared by the DNA preparation portion 11 as a template. Here, DNA derived from a subject's cells is amplified as an internal control. The DNA derived from the pathogen to be distinguished is (1) DNA derived from a pathogen colonizing the respiratory tract or (2) further includes DNA derived from a pathogen that is usually absent in the respiratory tract. In another embodiment, (3) the DNA derived from the pathogen to be distinguished further includes DNA corresponding to a region specific for a drug resistance gene. The DNA amplification means is preferably PCR, real-time PCR, or the like. The DNA detection portion 13 detects the DNA derived from the subject's cells and the pathogen-derived DNA, amplified by the DNA amplification means. The DNA detection can be performed simultaneously with or after the completion of the DNA amplification. Examples of the DNA detection method include intercalator method (SYBR Green method) and fluorescence probe method. For the DNA amplification and detection, it is preferable to use the combination of real-time PCR and fluorescence probe method.

The calculation portion 14 calculates (1) the relative number of a copy number of the pathogen-derived DNA to a copy number of the DNA derived from the subject's cells, based on a signal detected by the DNA detection means, when the pathogen to be distinguished is a pathogen colonizing the respiratory tract. Alternatively, it calculates (2) a copy number of the pathogen-derived DNA, when the pathogen to be distinguished is a pathogen that is usually absent in the respiratory tract. Then, the distinguishment portion 15 distinguishes (1) whether or not the pathogen colonizing the respiratory tract is the respiratory pathogen, based on the distinguishment reference value and the relative number. Specifically, the distinguishment is performed such that: i) when the DNA derived from the pathogen colonizing the respiratory tract is not detected, the pathogen is not colonizing the respiratory tract (negative); ii) when the DNA derived from the pathogen colonizing the respiratory tract is detected, albeit with the relative number below the distinguishment reference value, the pathogen is not the respiratory pathogen but a colonizing pathogen; and iii) when the relative number is equal to or above the distinguishment reference value, the pathogen is the respiratory pathogen. Alternatively, (2) when the pathogen to be distinguished is a pathogen that is usually absent in the respiratory tract, the distinguishment portion 15 performs distinguishment such that: i) when the DNA derived from the pathogen that is usually absent in the respiratory tract is not detected, the pathogen does not participate in the infection; and ii) when the DNA derived from the pathogen that is usually absent in the respiratory tract is detected, the pathogen is the respiratory pathogen.

### Examples

### (Example 1: DNA purification)

Samples used were any of the expectorated sputa, aspirated sputa, induced sputa, and intratracheally collected sputa of outpatients or hospitalized patients with respiratory infection. Moreover, sputa immediately after being expectorated or sputa cryopreserved at -20°C were used. To the sample, the equal amount of phosphate buffered saline (PBS) was added, and the mixture was vortexed to thereby obtain a uniform sample with reduced viscosity. To 200 µL of the sample, 200 µL of sample lysis buffer (Buffer AL, QIAGEN) and 20 µL of Proteinase K (TAKARA BIO INC.) were added, and the mixture was incubated at 57°C for 2 hours. Then, DNA in the sample was purified using QIAamp DNA Blood Mini Kit (QIAGEN).

### (Example 2: PCR reaction)

The purified DNA derived from each sample was used as a template to perform PCR reaction (real-time PCR) on 21 kinds of target genes. In this PCR reaction, Smart Cycler II System (TAKARA BIO INC.) was used, and one or two kinds of target genes were detected in one reaction. A PCR reaction solution and the PCR reaction were used under conditions shown below. The combinations of a primer set and a probe used for detecting 22 kinds of target genes are shown in Table 3. When two kinds of target nucleotide sequences were simultaneously amplified in one reaction, the combination of fluorescent labels differing between the probes therefor was used.

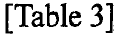

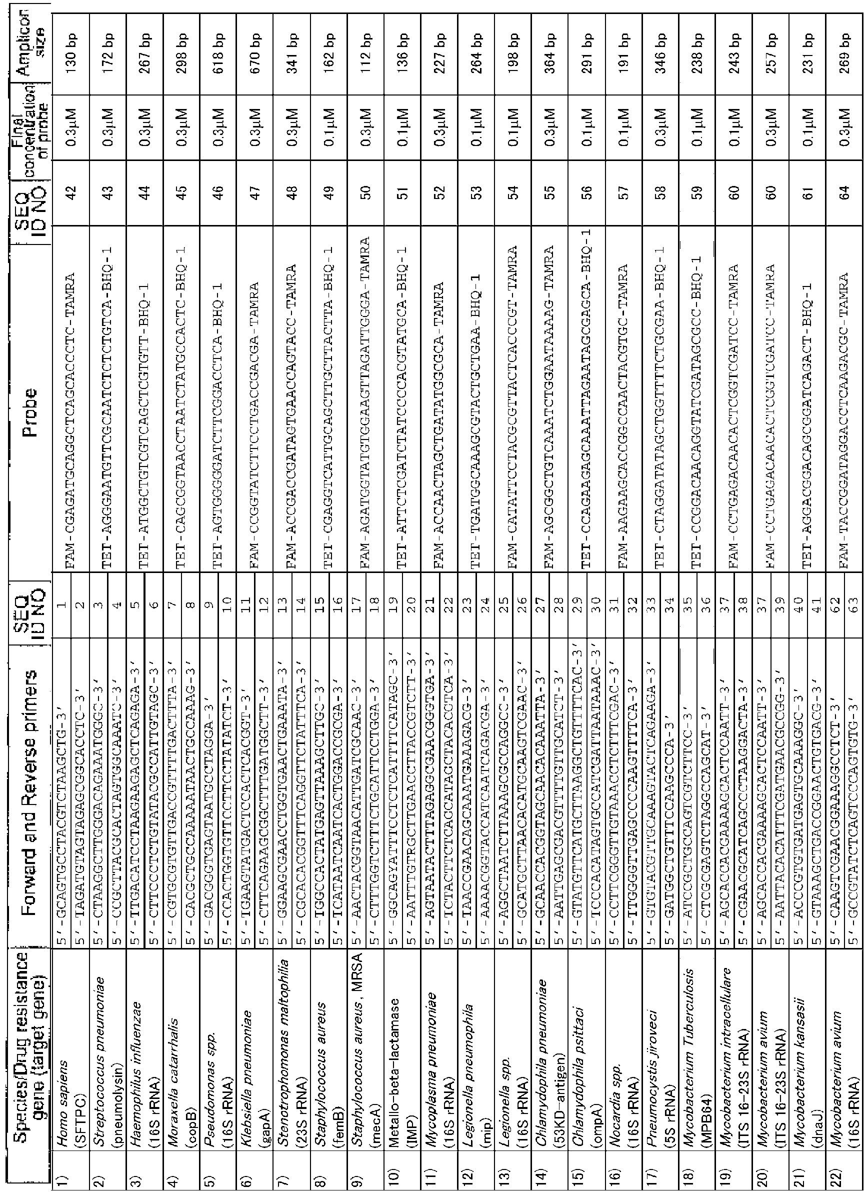

### [PCR reaction solution (single PCR)]

| | |
|---|---|
| TAKARA Premix Ex Taq | 12.5 µL |
| Target gene A forward primer (10 µM) | 0.75 µL |
| Target gene A reverse primer (10 µM) | 0.75 µL |
| Target gene A probe (10 µM) | 0.75 µL |
| Template DNA | 1.0 µL |
| Sterilized water | up to 25.0 µL |

### [PCR reaction solution (multiplex PCR)]

| | |
|---|---|
| TAKARA Premix Ex Taq | 12.5 µL |
| Target gene A forward primer (10 µM) | 0.75 µL |
| Target gene A reverse primer (10 µM) | 0.75 µL |
| Target gene A probe (10 µM) | 0.25 µL or 0.75 µL |
| Target gene B forward primer (10 µM) | 0.75 µL |
| Target gene B reverse primer (10 µM) | 0.75 µL |
| Target gene B probe (10 µM) | 0.25 µL or 0.75 µL |
| Template DNA | 1.0 µL |
| Sterilized water | up to 25.0 µL |

### [PCR reaction conditions (amplicon size: smaller than 500 bp)]

| | |
|---|---|
| First denaturation step | 95°C for 30 sec. |
| 3-step PCR (40 cycles) | |
| Denaturation step | 95°C for 8 sec. |
| Annealing step | 61°C for 25 sec. |
| Elongation step (fluorescence was detected) | 72°C for 20 |

sec.

### [PCR reaction conditions (amplicon size: equal to or larger than 500 bp)]

| | |
|---|---|
| First denaturation step | 95°C for 30 sec. |
| 3-step PCR (40 cycles) | |
| Denaturation step | 95°C for 10 sec. |
| Annealing step | 61°C for 35 sec. |
| Elongation step (fluorescence was detected) | 72°C for 25 sec. |

### (Example 3: Detection of pathogen in actual pneumonia cases)

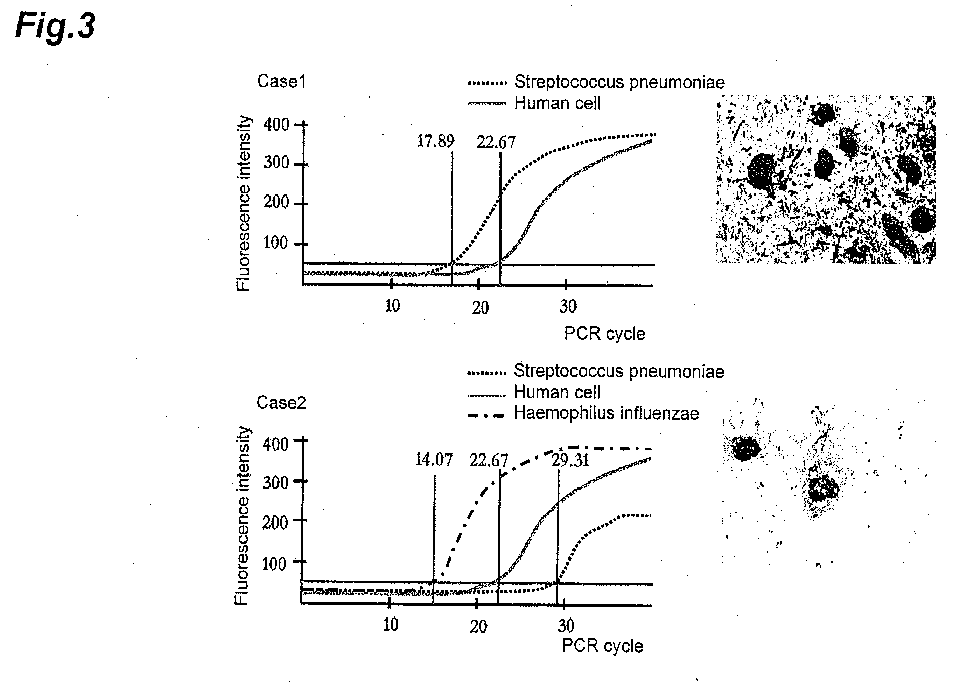

Results of PCR analysis of actual pneumonia cases (case 1 and case 2) are shown in Fig. 3. Fig. 3 is a graph showing the relationship between a PCR cycle number and fluorescence intensity in the detection of target genes, and a photograph of Gram-stained sputa. First, the Gram staining of sputa will be described. The photograph of Gram-stained sputa of case 1 is an actual photograph of case 1. A large number of human cells (leukocytes) are observed, while a larger number of streptococci (*Streptococcus pneumoniae*) stained dark blue are observed. The observations of the Gram-stained smear are probably derived from sputa with *Streptococcus pneumoniae* infection. Therefore, case 1 can be regarded as having pneumonia attributed to *Streptococcus pneugnoniae.* The photograph of Gram-stained sputa of case 2 is an actual photograph of case 2. Human cells (leukocytes) are observed, while a larger number of small bacilli (*Haemophilus influenzae)* stained red are observed. The observations of the Gram-stained smear are probably derived from sputa with *Haemophilus influenzae* infection. In the photograph of case 2, streptococci (*Streptococcus pneumoniae*) stained dark blue are also slightly observed but are much smaller in number than *Haemophilus influenzae,* and observations about suspected infection with this pathogen are not found. Therefore, *Streptococcus pneumoniae* of case 2 can be regarded as being a colonizing pathogen.

Results of detecting pathogen-specific genes in these samples of each case are shown in the graph of Fig. 3. In this graph, a gene whose fluorescence intensity is increased at a smaller PCR cycle number means a larger copy number of the DNA sequence in the sputa. Specifically, a cycle number (Cycle threshold; Ct value) at which fluorescence intensity reaches the predetermined value represents the relative copy number of the target gene (pathogen) in the sample. Moreover, a value (ΔCycle) determined by subtracting a Ct value of a certain pathogen from a Ct value of human cells as a control represents the relative amount of pathogen cells to the human cells as an internal control.

In the graph of case 1 (pneumonia attributed to *Streptococcus pneumoniae*), the dotted line represents an amplification curve of the *Streptococcus pneumoniae-specific* sequence, and the Ct value was 17.89 cycles. The solid line represents an amplification curve of the human cell-specific sequence, and the Ct value was 22.67 cycles. Thus, ΔCycle of *Streptococcus pneumoniae* was demonstrated to be +4.78 cycles.

In case 2, the Ct value of an amplification curve of the *Streptococcus pneumoniae-specific* sequence (dotted line) was 29.31 cycles, and the Ct value of an amplification curve of the human cell-specific sequence (solid line) was 22.67 cycles. Thus, ΔCycle of *Streptococcus pneumoniae* was -6.64 cycles. On the other hand, the Ct value and ΔCycle of an amplification curve of the *Haemophilus influenzae-*specific sequence were 14.07 cycles and +6.60 cycles, respectively. From this, the relative copy number of the *Haemophilus influenzae*-specific sequence can be determined to be significantly higher in case 2 than that of *Streptococcus pneumoniae.* This result correlates with the observations obtained by Gram staining of sputum smear. Moreover, with regard to *Streptococcus pneumoniae,* ΔCycle of *Streptococcus pneumoniae* was demonstrated to be significantly higher in case 1 than in case 2. This result correlates with the diagnosis results of infection attributed/unattributed to *Streptococcus pneumoniae.*

Thus, it was demonstrated that the relative number (ΔCycle) of pathogen cells to the number of human cells as an internal control can be converted into numbers.

### (Examples 4 to 9: Setting of distinguishment reference values (cutoff values) in actual pneumonia cases)

PCR analysis was conducted by the procedures shown in Examples 1 and 2 using clinical samples of actual pneumonia cases to evaluate the usefulness of the method of distinguishing a respiratory pathogen. Two analyses were conducted using clinical samples (first: 207 cases or 300 cases, second: 223 cases) overlapping between the first and second analyses. In the first analysis, the clinical samples used were only sputa obtained from outpatients or hospitalized patients within 3 days from consultation. In the second analysis, the criteria of the clinical samples to be analyzed were more strictly set for the analysis. Specifically, the clinical samples used were sputa, intratracheally collected sputa, or induced sputa obtained from outpatients or hospitalized patients within 2 days from consultation. A *Mycobacterium avium* 16-23 ITS rRNA gene was detected as a *Mycobacterium avium*-specific gene.

### (Example 4: Setting of distinguishment reference value of Streptococcus pneumoniae)

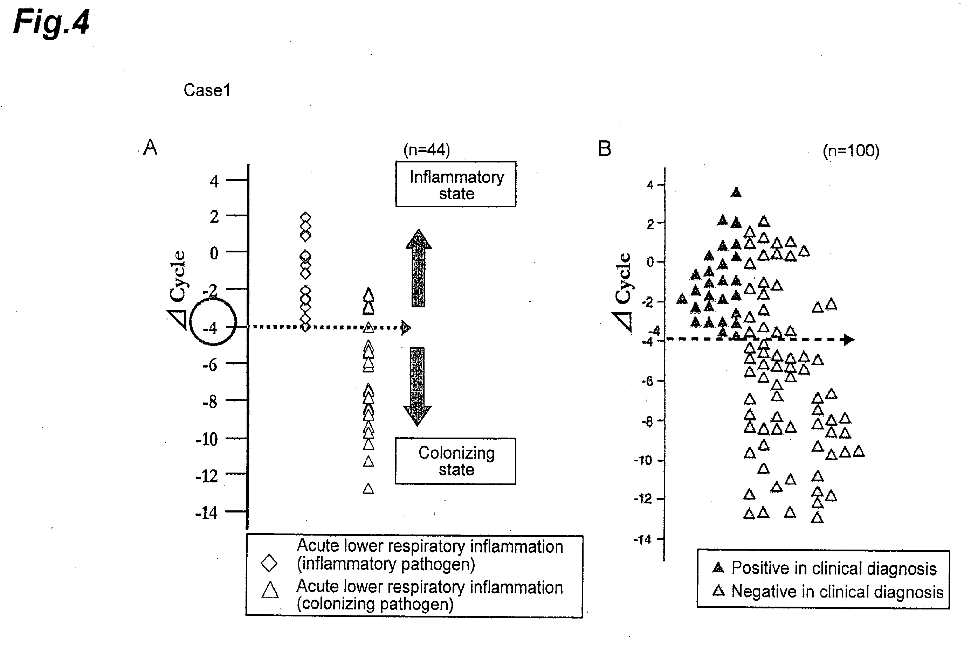

(4-1) Of 207 cases, 44 cases from which *Streptococcus pneumoniae* was detected by PCR are shown in Fig. 4A. Based on these 44 cases, a distinguishment reference value was set. Fig. 4A is a plot based on ΔCycle obtained in each of 15 cases (rhombus) clinically diagnosed as having *Streptococcus pneumoniae*-induced pneumonia and 29 cases of pneumonia (triangle) that could not be clinically diagnosed as being *Streptococcus pneumoniae*-induced pneumonia or was diagnosed as being attributed to other respiratory pathogens. From these results, the lower limit of ΔCycle leading to respiratory pathogen positive was determined, and the distinguishment reference value of inflammation/colonization for *Streptococcus pneumoniae* was set to "-4".

(4-2) Of 223 cases, 100 cases from which *Streptococcus pneumoniae* was detected by PCR are shown in Fig. 4B. Based on 27 cases out thereof in which *Streptococcus pneumoniae* was a respiratory pathogen in the existing method, a distinguishment reference value was set. Fig. 4B is a plot based on ΔCycle obtained in each of 27 cases (filled triangle) clinically diagnosed as having *Streptococcus pneumoniae*-induced pneumonia and 73 cases (open triangle) of pneumonia that could not be clinically diagnosed as being *Streptococcus pneumoniae*-induced pneumonia though *Streptococcus pneumoniae* was detected by PCR or pneumonia that was diagnosed as being attributed to other respiratory pathogens. From these results, the lower limit of ΔCycle leading to respiratory pathogen positive was determined, and the distinguishment reference value of inflammation/colonization for *Streptococcus pneumoniae* was set to "-4".

(Example 5: Setting of distinguishment reference value of *Haemophilus influenzae*)

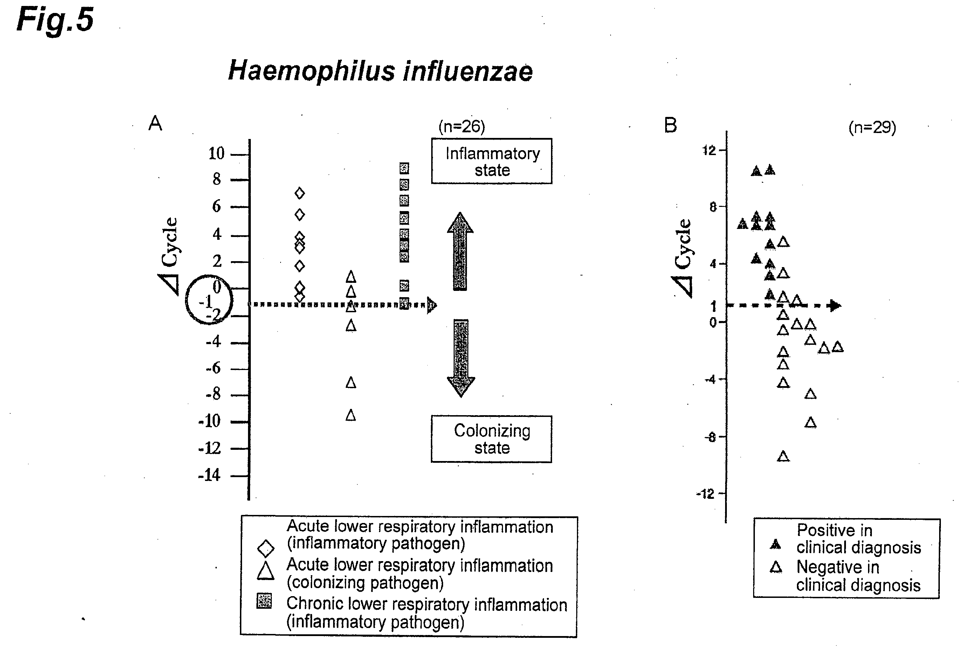

(5-1) Of 207 cases, 26 cases from which *Haemophilus influenzae* was detected by PCR are shown in Fig. 5A. Based on these 26 cases, a distinguishment reference value was set. Fig. 5A is a plot based on ΔCycle obtained in each of 9 cases of acute respiratory infection (rhombus) in which *Haemophilus influenzae* was clinically diagnosed as being a respiratory pathogen, 6 cases of acute respiratory infection (triangle) in which *Haemophilus influenzae* was regarded as being a colonizing pathogen, and 11 cases of chronic respiratory infection (square) in which *Haemophilus influenzae* was clinically diagnosed as being a respiratory pathogen. From these results, the lower limit of ΔCycle leading to respiratory pathogen positive was determined, and the distinguishment reference value of inflammation/colonization for *Haemophilus influenzae* was set to "-1".

(5-2) Of 223 cases, 29 cases from which *Haemophilus influenzae* was detected by PCR are shown in Fig. 5B. Based on 12 cases out thereof in which *Haemophilus influenzae* was a respiratory pathogen in the existing method, a distinguishment reference value was set. Fig. 5B is a plot based on ΔCycle obtained in each of 12 (filled triangle), out of the 223 pneumonia cases, in which *Haemophilus influenzae* was clinically diagnosed as being a respiratory pathogen, and 17 cases (open triangle) that could not be diagnosed as being *Haemophilus influenzae*-induced pneumonia though *Haemophilus influenzae* was detected by PCR (i.e., *Haemophilus influenzae* was regarded as being a colonizing pathogen). From these results, the lower limit of ΔCycle leading to respiratory pathogen positive was determined, and the distinguishment reference value of inflammation/colonization for *Haemophilus influenzae* was set to "1". Thus, a more highly reliable distinguishment reference value can be set by analyzing an increased number of cases in which ΔCycle obtained by the present invention can be compared with the existing diagnosis results.

### (Example 6: Setting of distinguishment reference value of Pseudomonas aeruginosa)

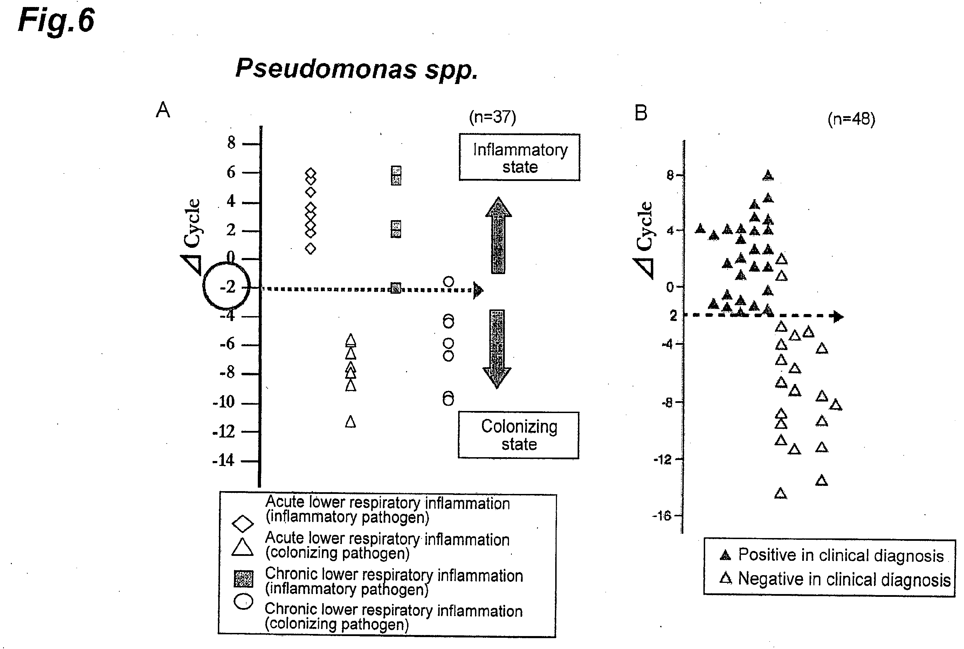

(6-1) Of 207 cases, 37 cases from which *Pseudomonas aeruginosa* was detected by PCR are shown in Fig. 6A. Based on these 37 cases, a distinguishment reference value was set. Fig. 6A is a plot based on ΔCycle obtained in each of 8 cases of acute respiratory infection (rhombus) in which *Pseudomonas aeruginosa* was clinically diagnosed as being a respiratory pathogen, 7 cases of acute respiratory infection (triangle) in which *Pseudomonas aeruginosa* was regarded as being a colonizing pathogen, 15 cases of chronic respiratory infection (square) in which *Pseudomonas aeruginosa* was clinically diagnosed as being a respiratory pathogen, and 7 cases of chronic respiratory infection (circle) in which *Pseudomonas aeruginosa* was regarded as being a colonizing pathogen. From these results, ΔCycle serving as a respiratory pathogen/colonizing pathogen threshold was determined, and the distinguishment reference value of inflammation/colonization for the bacterium of the genus *Pseudomonas* was set to "-2".

(6-2) Of 223 cases, 48 cases from which *Pseudomonas aeruginosa* was detected by PCR are shown in Fig. 6B. Based on 27 cases out thereof in which *Pseudomonas aeruginosa* was a respiratory pathogen in the existing method, a distinguishment reference value was set. Fig. 6B is a plot based on ΔCycle obtained in each of 27 (filled triangle), out of the 223 pneumonia cases, in which *Pseudomonas aeruginosa* was clinically diagnosed as being a respiratory pathogen and 21 cases (open triangle) that could not be diagnosed as being *Pseudomonas aeruginosa*-induced pneumonia though *Pseudomonas aeruginosa* was detected by PCR (i.e., *Pseudomonas aeruginosa* was regarded as being a colonizing pathogen). From these results, the lower limit of ΔCycle leading to respiratory pathogen positive was determined, and the distinguishment reference value of inflammation/colonization for *Pseudomonas aeruginosa* was set to "-2".

### (Example 7: Setting of distinguishment reference value of Moraxella catarrhalis)

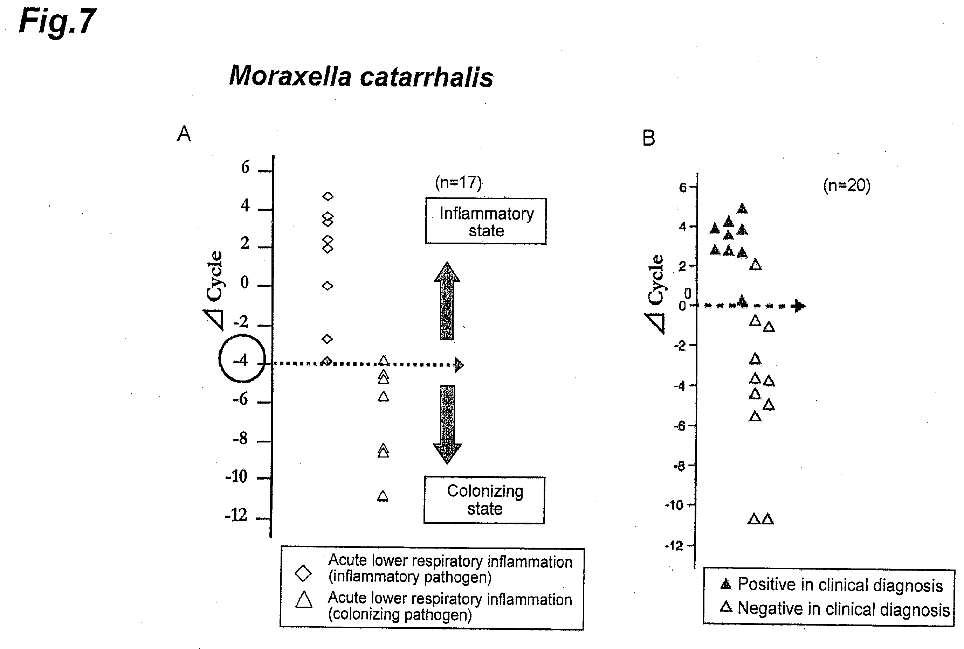

(7-1) Of 300 cases, 17 cases from which *Moraxella catarrhalis* was detected by PCR are shown in Fig. 7A. Based on these 17 cases, a distinguishment reference value was set. Fig. 7A is a plot based on ΔCycle obtained in each of 9 cases of acute respiratory infection (rhombus) in which *Moraxella catarrhalis* was clinically diagnosed as being a respiratory pathogen and 8 cases of acute respiratory infection (triangle) in which *Moraxella catarrhalis* was regarded as being a colonizing pathogen. From these results, ΔCycle serving as a respiratory pathogen/colonizing pathogen threshold was determined, and the distinguishment reference value of inflammation/colonization for *Moraxella catarrhalis* was set to "-4".

(7-2) Of 223 cases, 20 cases from which *Moraxella catarrhalis* was detected by PCR are shown in Fig. 7B. Based on 9 cases out thereof in which *Moraxella catarrhalis* was a respiratory pathogen in the existing method, a distinguishment reference value was set. Fig. 7B is a plot based on ΔCycle obtained in each of 9 (filled triangle), out of the 223 pneumonia cases, in which *Moraxella catarrhalis* was clinically diagnosed as being a respiratory pathogen and 11 cases (open triangle) that could not be diagnosed as being *Moraxella catarrhalis-*induced pneumonia though *Moraxella catarrhalis* was detected by PCR (i.e., *Moraxella catarrhalis* was regarded as being a colonizing pathogen). From these results, the lower limit of ΔCycle leading to respiratory pathogen positive was determined, and the distinguishment reference value of inflammation/colonization for *Moraxella catarrhalis* was set to "0".

### (Example 8: Setting of distinguishment reference value of methicillin-resistant Staphylococcus aureus)

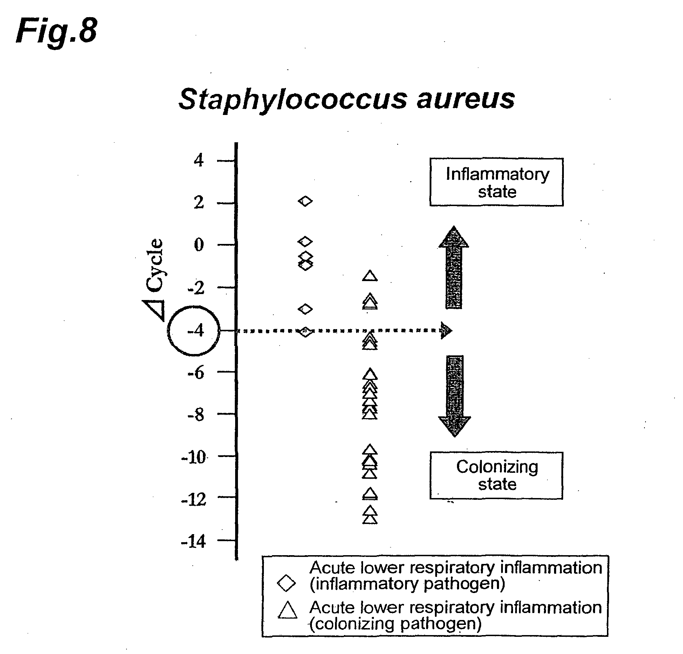

(8-1) Of 300 cases, 34 cases from which methicillin-resistant *Staphylococcus aureus* (hereinafter, MRSA) was detected by PCR are shown in Fig. 8. Based on these 34 cases, a distinguishment reference value was set. Fig. 8 is a plot based on ΔCycle obtained in each of 8 cases of acute respiratory infection (rhombus) in which MRSA was clinically diagnosed as being a respiratory pathogen and 26 cases of acute respiratory infection (triangle) in which MRSA was regarded as being a colonizing pathogen. From these results, the lower limit of ΔCycle leading to respiratory pathogen positive was determined, and the distinguishment reference value of inflammation/colonization for MRSA was set to "-4".

(8-2) Based on cases, out of 223 cases, in which MRSA was a respiratory pathogen in the existing method, a distinguishment reference value was set in the same way as in Examples 4 to 8. Based on ΔCycle obtained in each case, the lower limit of ΔCycle leading to respiratory pathogen positive was determined, and the distinguishment reference value of inflammation/colonization for MRSA was set to "-4".

### (Example 9: Setting of distinguishment reference value of Klebsiella pneumoniae)

Based on cases, out of 223 cases, in which *Klebsiella pneumoniae* was a respiratory pathogen in the existing method, a distinguishment reference value was set in the same way as in Examples 4 to 8 in the second evaluation. Based on ΔCycle obtained in each case, the lower limit of ΔCycle leading to respiratory pathogen positive was determined, and the distinguishment reference value of inflammation/colonization for *Klebsiella pneumoniae* was set to "-4" (not shown).

A distinguishment reference value can be set for every pathogen colonizing the respiratory tract, in the same way as in Examples 4 to 9. Moreover, the number of cases can be added to thereby enhance the reliability of the set distinguishment reference value and set a more highly reliable distinguishment reference value.

### (Example 10: Example of practical use in actual pneumonia cases)

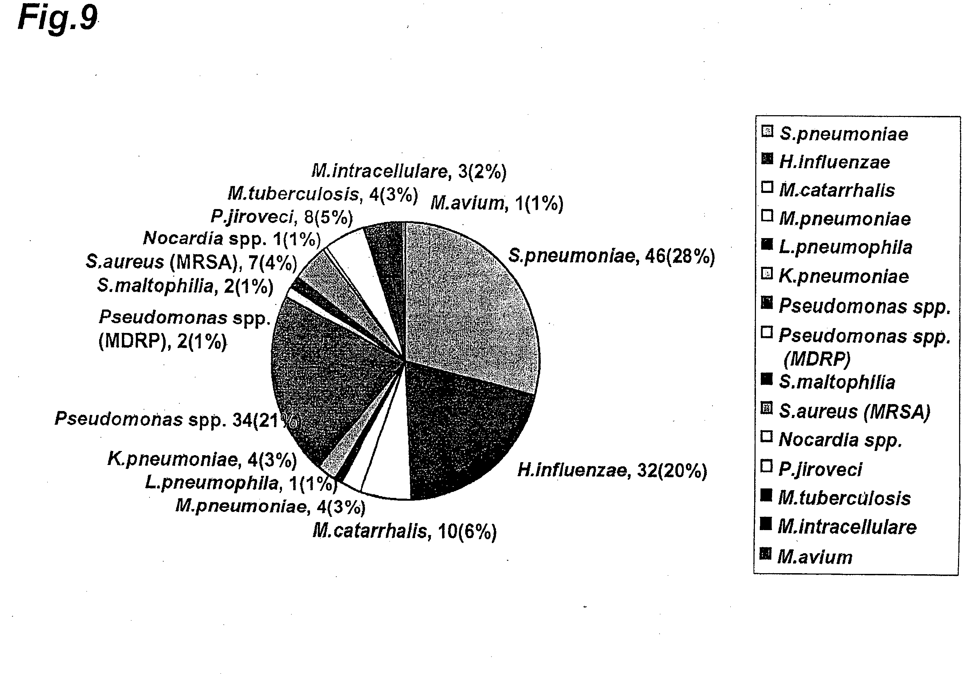

The clinical samples of actual pneumonia cases (300 cases overlapping with the pneumonia cases of Examples 4 to 8) used in the first analysis were subjected to the PCR analysis described above to identify a respiratory pathogen based on the distinguishment reference values set in Examples 4 to 8. The results are shown in Fig. 9. The rate of pathogen detection was 236 out of the 300 cases (78.7%). Moreover, the respiratory pathogen was identified in 158 cases among the 300 cases. The rate of respiratory pathogen identification by detection of 20 kinds of pathogens/drug resistance genes except for the control human gene was 52.7%.

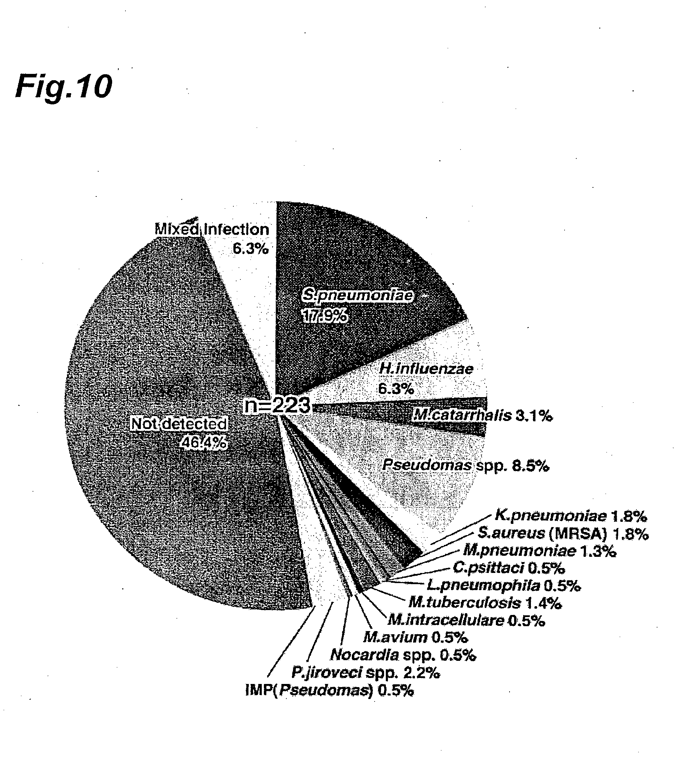

The clinical samples of actual pneumonia cases (223 cases) used in the second analysis were subjected to the PCR analysis described above to identify a respiratory pathogen based on the distinguishment reference values set in Examples 4 to 9. The results are shown in Fig. 10. The rate of respiratory pathogen detection in the 223 pneumonia cases was 119 out of the 223 cases (53.36%). Thus, it could be confirmed that a very high identification rate is obtained as rapid test results obtained from one sputum sample and one series of test.

Thus, it was demonstrated that the distinguishment of the respiratory pathogen by PCR analysis with human cells as an internal control effectively functions owing to the distinguishment reference value set based on ΔCycle. This detection method is effective particularly for detecting a pathogen colonizing the respiratory tract, i.e., distinguishing between the respiratory pathogen and a colonizing pathogen. Moreover, the multiplex PCR that detects human cells and various pathogens/drug resistance genes (a total of 21 kinds or 22 kinds) shown in Table 3 achieved comprehensive search of pathogens colonizing the respiratory tract, pathogens that are usually absent in the respiratory tract, etc., by one test. As a result, a respiratory pathogen as a main factor of infection can be identified, and efficient treatment such as drug treatment suitable for individual pathogens can be performed.

### (Example 11: Prospective trial)

To study the validity of the distinguishment reference values set in Examples 4 to 9, clinical trial was conducted (UMIN trial ID: UMIN000001118). This trial was multicenter prospective trial involving repeatedly enrolled pneumonia cases, by which a method of identifying a respiratory pathogen by PCR analysis using the distinguishment reference value set in the second analysis was compared with a method of identifying a respiratory pathogen using the existing method. The main outcome evaluation item is to study the validity of the distinguishment reference value for *Streptococcus pneumoniae-induced* pneumonia. A *Mycobacterium avium* 16S rRNA gene was detected as a *Mycobacterium* avium-specific gene.

Cases that satisfied the following criteria of case selection were adopted as test subjects:
cases in 18-old-year or older subjects from which sputa were obtained within 2 days after consultation or the onset of pneumonia;
cases in which sputa were included in M2, P1, P2, P3 according to the Miller-Jones classification and the Ct value of a human cell-specific sequence was below 27; and
cases on which sputum test (smear/culture) and *Streptococcus pneumoniae* urinary antigen test had been conduced.
Moreover, pneumonia handled here was acute respiratory infection (acute pneumonia) and was limited to those in which the new emergence of a shadow was observed in the diagnostic imaging of the chest, along with the new emergence of respiratory symptoms (fever, sputa, cough, pleurodynia, and dyspnea).

For pathogens colonizing the respiratory tract, the definition of diagnosis of a respiratory pathogen by the existing clinical diagnosis is as follows:
of pathogens colonizing the respiratory tract, detected in PCR, those taking a therapeutic cause compatible as a respiratory pathogen and satisfying any of the following (1) to (4):
   (1) such pathogens are detected from sterile samples (e.g., blood culture or pleural effusion culture);
   (2) a pathogen compatible with such pathogens is confirmed in Gram staining, and furthermore, such pathogens are detected/identified in sputum culture;
   (3) a large number of leukocytes (>25 cells/field at x100) are observed in Gram staining, and furthermore, a pathogen morphologically compatible with such pathogens is gormandized; and
   (4) positive for *Streptococcus pneumoniae* urinary antigens (this condition holds true only for *Streptococcus pneumoniae*).

As a result of multicenter prospective trial, 174 cases of pneumonia were enrolled. Of them, 43 cases were cases in which *Streptococcus pneumoniae* was identified as a respiratory pathogen in the existing method. On the other hand, 40 cases were cases in which *Streptococcus pneumoniae* was identified as a respiratory pathogen by PCR analysis using the distinguishment reference value described above. The rate (sensitivity) of respiratory bacterium identification in *Streptococcus pneumoniae*-induced pneumonia was 93.02% (40/43; 95% confidence interval: 0.83<) (the identification method using the cutoff value/the identification method using the existing method). Moreover, the ability to deny that *Streptococcus pneumoniae,* if any, detected by PCR was a respiratory pathogen (negative predictive value) was 93.48%. For other pathogens, preliminary data that produced equivalent or higher reliable results were obtained.

From these results, the following (1) to (4) could be confirmed, particularly for *Streptococcus pneumoniae:*
(1) the distinguishment method of the present invention can highly sensitively distinguish cases that can be diagnosed by the existing diagnosis method (the positive population in the distinguishment method of the present invention encompasses a positive population in the existing test);
(2) the positive population in the distinguishment method of the present invention has a *Streptococcus pneumoniae*/subject-derived cell ratio (i.e., ΔCycle value) equivalent to a positive population in the existing diagnosis method;
(3) thus, it was demonstrated that the positive population in the distinguishment method of the present invention can be considered as a group to be treated (i.e., a group in which *Streptococcus pneumoniae* should be estimated to be a respiratory pathogen); and
(4) the therapeutic course of cases in prospective clinical trial also supported the validity of results of distinguishing *Streptococcus pneumoniae* to be treated, based on the distinguishment results of the present invention.

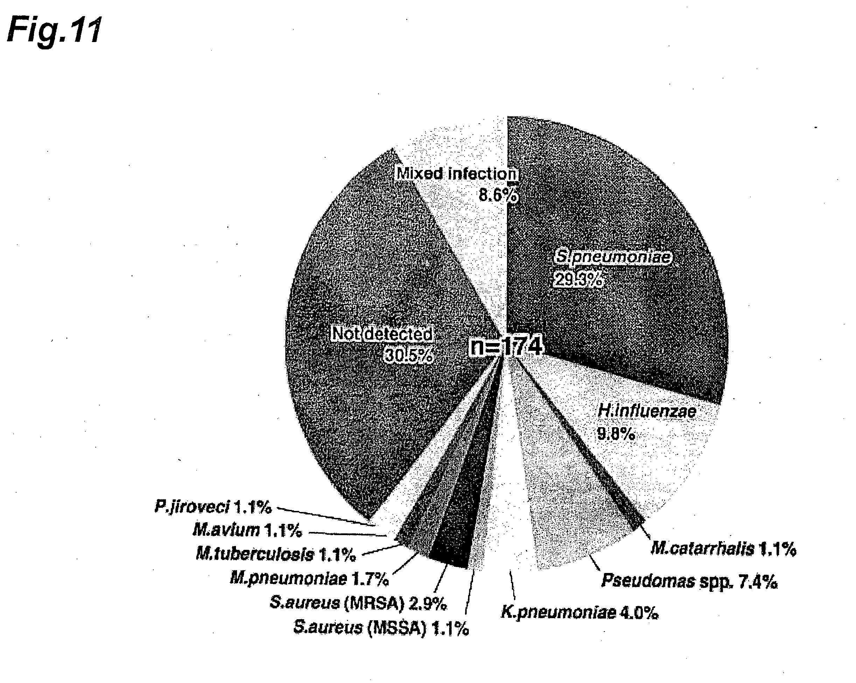

For the clinical samples of 174 pneumonia cases enrolled in clinical trial, a respiratory pathogen was identified by PCR analysis using the distinguishment reference values. The results are shown in Fig. 11. The rate of respiratory pathogen identification in the 174 pneumonia cases was 121 out of the 174 cases (70%) (Fig. 11). The rate of respiratory pathogen identification using the existing method was 97 out of the 174 cases (55%). Thus, it was demonstrated that the distinguishment method of the present invention can identify a respiratory pathogen more rapidly and has a higher detection rate, than the existing method.

## Claims

1. A method of distinguishing a respiratory pathogen causing acute respiratory infection, wherein a pathogen to be distinguished includes a pathogen colonizing the respiratory tract, the method comprising:
measuring the relative value of the amount of the pathogen in a sample containing the respiratory secretions of a subject, to the amount of the subject's cells in the sample; and
distinguishing whether or not the pathogen is the respiratory pathogen, based on the relative value.

2. A method of determining a distinguishment reference value for distinguishing a respiratory pathogen causing acute respiratory infection, wherein a pathogen to be distinguished is a pathogen colonizing the respiratory tract, the method comprising:
a) measuring a gene copy number derived from a subject's cells, in DNA prepared from a sample containing the respiratory secretions of the subject;
b) measuring a pathogen-derived gene copy number in the DNA;
c) calculating the relative number of the pathogen-derived gene copy number to the gene copy number derived from the subject's cells;
d) performing the a) to c) on a plurality of subjects to obtain the relative number for each of the subjects; and
e) comparing the relative number of each of the subjects with clinical diagnosis results of each of the subjects to thereby determine, as a distinguishment reference value:
i) the lower limit of the relative number leading to respiratory pathogen positive in clinical diagnosis; and/or
ii) the upper limit of the relative number leading to respiratory pathogen negative in clinical diagnosis.

3. A method of distinguishing a respiratory pathogen causing acute respiratory infection, wherein a pathogen to be distinguished is a pathogen colonizing the respiratory tract, the method comprising:
a) measuring a gene copy number derived from a subject's cells, in DNA prepared from a sample containing the respiratory secretions of the subject;
b) measuring a pathogen-derived gene copy number in the DNA;
c) calculating the relative number of the pathogen-derived gene copy number to the gene copy number derived from the subject's cells; and
d) distinguishing whether or not the pathogen is the respiratory pathogen, based on the distinguishement reference value according to claim 2 and the relative number.

4. The method according to claim 2 or 3, wherein the pathogen colonizing the respiratory tract is one or more pathogens selected from the group consisting of microbes of the genera *Streptococcus, Haemophilus, Moraxella, Pseudomonas, Klebsiella, Stenotrophomonas, Acinetobacter,* and *Staphylococcus.*

5. The method according to claim 3 or 4, wherein an additional pathogen to be distinguished is a pathogen that is usually absent in the respiratory tract, the method further comprising:
a) measuring a pathogen-derived gene copy number in DNA prepared from a sample containing the respiratory secretions of a subject; and
b) performing the distinguishment such that:
i) when the pathogen-derived gene is not detected, the pathogen does not participate in the infection; and
ii) when the pathogen-derived gene is detected, the pathogen is the respiratory pathogen.

6. The method according to claim 5, wherein the pathogen that is usually absent in the respiratory tract is one or more pathogens selected from the group consisting of microbes of the genera *Mycoplasma, Legionella, Chlamydophila, Mycobacterium, Coxiella, Nocardia, Pneumocystis,* and *Aspergillus.*

7. The method according to any one of claims 2 to 6, wherein the pathogen-derived gene is one or more genes derived from a pathogen colonizing the respiratory tract, selected from the group consisting of a *Streptococcus pneumonia* pneumolysin gene, a *Streptococcus pneumonia* lytA gene, a *Haemophilus influenza* 16S rRNA gene, a *Moraxella catarrhalis* copB gene, a *Pseudomonas* 16S rRNA gene, a *Klebsiella pneumonia* gapA gene, a *Stenotrophomonas maltophilia* 23S sRNA gene, and a *Staphylococcus aureus* femB gen.

8. The method according to any one of claims 5 to 7, wherein the pathogen-derived gene is one or more genes derived from a pathogen that is usually absent in the respiratory tract, selected from the group consisting of *Mycoplasma pneumonia* 16S rRNA gene, a *Legionella pneumophila* mip gene, a *Legionella* 16S rRNA gene, a *Chlamydophila pneumonia* 53-kD antigen gene, a *Chlamydophila psittaci* ompA gene, a *Mycobacterium tuberculosis* MPB64 gene, a *Mycobacterium intracelulare* ITS 16-23S rRNA gene, a *Mycobacterium avium* 16S rRNA gene, a *Mycobacterium avium* ITS 16-23S rRNA gene, a *Mycobacterium kansassi* dnaJ gene, a *Nocardia* 16S rRNA gene, and a *Pneumocystis jiroveci* 5S rRNA gene.

9. The method according to any one of claims 2 to 8, wherein the pathogen-derived gene further comprises a drug resistance gene.

10. The method according to claim 9, wherein the drug resistance gene is one or more genes selected from the group consisting of a mecA gene and a metallo-β-lactamase gene.

11. The method according to any one of claims 1 to 10, wherein the subject is a human.

12. Use of a kit for performing a method according to any one of claims 1 to 11, said kit comprising: a primer set for a gene derived from one or more pathogens colonizing the respiratory tract, selected from the group consisting of microbes of the genera *Streptococcus, Haemophilus, Moraxella, Pseudomonas, Klebsiella, Stenotrophomonas, Acinetobacter,* and *Staphylococcus*; and a manual.

13. Use according to claim 12, wherein the kit further comprises a primer set for a gene derived from one or more pathogens that are usually absent in the respiratory tract, selected from the group consisting of microbes of the genera *Mycoplasma, Legionella, Chlamydophila, Mycobacterium, Coxiella, Nocardia, Pneumocystis,* and *Aspergillus;* and a manual.

14. Use according to claim 12 or 13, wherein the kit further comprises: a primer set for one or more drug resistance genes selected from the group consisting of a mecA gene and a metallo-β-lactamase gene; and a manual.

15. Use of an apparatus for performing a method according to any one of claims 1 to 11, the apparatus comprising:
a) DNA preparation means for preparing DNA from a sample containing the respiratory secretions of a subject;
b) DNA amplification means for specifically amplifying DNA derived from the subject's cells and pathogen-derived DNA, in the DNA;
c) DNA detection means for detecting the DNA derived from the subject's cells and the pathogen-derived DNA, amplified by the DNA amplification means;
d) calculation means for calculating the relative number of a copy number of the pathogen-derived DNA to a copy number of the DNA derived from the subject's cells, based on a signal detected by the DNA detection means; and
e) distinguishment means for distinguishing whether or not the pathogen is the respiratory pathogen, based on the distinguishment reference value according to claim 2 and the relative number.

16. The use according to claim 15, wherein an additional pathogen to be distinguished is a pathogen that is usually absent in the respiratory tract, the apparatus further comprising:
a) DNA preparation means for preparing DNA from a sample containing the respiratory secretions of a subject;
b) DNA amplification means for specifically amplifying a pathogen-derived DNA in the DNA;
c) DNA detection means for detecting the pathogen-derived DNA amplified by the DNA amplification means; and
d) distinguishment means for performing distinguishment such that:
i) when the pathogen-derived gene is not detected, the pathogen does not participate in the infection; and
ii) when the pathogen-derived gene is detected, the pathogen is the respiratory pathogen.

## Patentansprüche

1. Verfahren zum Erkennen eines respiratorischen Pathogens, welches akute Infektion der Atemwege verursacht, wobei ein zu erkennendes Pathogen ein Pathogen umfasst, welches die Atemwege kolonisiert, wobei das Verfahren umfasst:
Messen des relativen Werts der Menge des Pathogens in einer Probe, welche die respiratorischen Sekrete eines Subjekts enthält, gegenüber der Menge der Zellen des Subjekts in der Probe; und
Erkennen ob das Pathogen das respiratorische Pathogen ist oder nicht, basierend auf dem relativen Wert.

2. Verfahren zum Bestimmen eines Erkennungsreferenzwerts zum Erkennen eines respiratorischen Pathogens, welches akute Infektion der Atemwege verursacht, wobei ein zu erkennendes Pathogen ein Pathogen umfasst, welches die Atemwege kolonisiert, wobei das Verfahren umfasst:
a) Messen einer Genkopieanzahl, welche von Zellen eines Subjekts abstammt, in DNA, welche aus einer Probe, welche die respiratorischen Sekrete des Subjekts enthalten, hergestellt wird;
b) Messen einer Genkopieanzahl, die von Pathogen abstammt, in der DNA;
c) Berechnen der relativen Anzahl der Genkopieanzahl, die von Pathogen abstammt, zu der Genkopieanzahl, welche von den Zellen eines Subjekts abstammt;
d) Durchführen von a) bis c) an einer Vielzahl von Subjekten, um die relative Anzahl für jedes der Subjekte zu erhalten; und
e) Vergleichen der relativen Anzahl von jeglichen Subjekten mit den klinischen Diagnoserergebnissen von jeglichen Subjekten, um dadurch einen Erkennungsreferenzwert zu bestimmen:
i) die untere Grenze der relativen Anzahl hat respiratorisches Pathogen positiv in klinischer Diagnose zur Folge; und/oder
ii) die obere Grenze der relativen Anzahl hat respiratorisches Pathogen negativ in klinischer Diagnose zur Folge.

3. Verfahren zum Erkennen eines respiratorischen Pathogens, welches akute Infektion der Atemwege verursacht, wobei ein zu erkennendes Pathogen ein Pathogen ist, welches die Atemwege kolonisiert, wobei das Verfahren umfasst:
a) Messen einer Genkopieanzahl, welche von Zellen eines Subjekts abstammt, in DNA, welche von einer Probe, welche die respiratorischen Sekrete des Subjekts enthalten, hergestellt wird;
b) Messen einer pathogen-abgeleiteten Genkopieanzahl in der DNA;
c) Berechnen der relativen Anzahl Genkopieanzahl, die von Pathogen abstammt, zu der Genkopieanzahl, welche von den Zellen eines Subjekts abstammt; und
d) Erkennen, ob das Pathogen das respiratorische Pathogen ist oder nicht, basierend auf dem Erkennungsreferenzwert nach Anspruch 2 und der relativen Anzahl.

4. Verfahren nach Anspruch 2 oder 3, wobei das Pathogen, welches die Atemwege kolonisiert ein oder mehrere Pathogene sind ausgewählt aus der Gruppe bestehend aus Mikroben der Gattungen *Streptococcus, Haemophilus, Moraxella, Pseudomonas, Klebsilla, Stenotrophomonas, Acinetobacter* und *Staphylococcus.*

5. Verfahren nach Anspruch 3 oder 4, wobei ein zusätzliches zu erkennendes Pathogen ein Pathogen ist, welches normalerweise in den Atemwegen abwesend ist, wobei das Verfahren umfasst:
a) Messen einer Genkopieanzahl, die von Pathogen abstammt, in DNA, welche von einer Probe hergestellt wird, welche die respiratorischen Sekrete eines Subjekts enthält; und
b) Durchführen der Erkennung, so dass:
i) wenn das pathogen-abgeleitete Gen nicht detektiert wird, das Pathogen nicht an der Infektion teilnimmt; und
ii) wenn das pathogen-abgeleitete Gen detektiert wird, das Pathogen ein respiratorisches Pathogen ist.

6. Verfahren nach Anspruch 5, wobei das Pathogen, welches normalerweise in den Atemwegen abwesend ist ein oder mehrere Pathogene ist ausgewählt aus der Gruppe bestehend aus Mikroben der Gattungen *Mycoplasma, Legionella, Chlamydophila, Mycobacterium, Coxiella, Nocarida, Pneumocystis* und *Aspergillus.*

7. Verfahren nach einem der Ansprüche 2 bis 6, wobei das pathogen-abgeleitete Gen ein oder mehrere Gene ist, welche von einem Pathogen, welches die Atemwege kolonisiert, abstammt, ausgewählt aus der Gruppe bestehend aus einem *Streptococcus pneumonia* pneumolysin-Gen, einem *Streptococcus pneumonia* lytA-Gen, einem *Haemophilus influenza* 16S rRNA-Gen, einem *Moraxella catarrhalis* copB-Gen, einem *Pseudomonas* 16S rRNA-Gen, einem *Klebsiella pneumonia* gapA-Gen, einem *Stenorophomonas maltophilia* 23S sRNA-Gen und einem *Staphylococcus aureus* femB-Gen.

8. Verfahren nach einem der Ansprüche 5 bis 7, wobei das Gen, das von Pathogen abstammt, ein oder mehrere Gene ist, welche von einem Pathogen abstammen, welches normalerweise in den Atemwegen abwesend ist, ausgewählt aus der Gruppe bestehend aus *Mycoplasma pneumonia* 16S rRNA-Gen, einem *Legionella pneumophila* mip-Gen, einem *Legionella* 16S rRNA-Gen, einem *Chlamydophila pneumonia* 53-kD Antigen-Gen, einem *Chlamydophila psittaci* ompA-Gen, einem *Mycobacterium tuberculosis* MPB64-Gen, einem *Mycobacterium intracelulare* ITS 16-23S rRNA-Gen, einem *Mycobacterium avium* 16S rRNA-Gen, einem *Mycobacterium avium* ITS 16-23S rRNA-Gen, einem *Mycobacterium kansassi* dnaJ-Gen, einem *Nocardia* 16S rRNA-Gen und einem *Pneumocystis jiroveci* 5S rRNA-Gen.

9. Verfahren nach einem der Ansprüche 2 bis 8, wobei das pathogenstammende Gen weiterhin ein Arzneimittel-resistentes Gen umfasst.

10. Verfahren nach Anspruch 9, wobei das Arzneimittel-resistente Gen ein oder mehrere Gene ist, ausgewählt aus der Gruppe bestehend aus einem mecA-Gen und einem metallo-β-lactamase-Gen.

11. Verfahren nach einem der Ansprüche 1 bis 10, wobei das Subjekt ein Mensch ist.

12. Verwendung eines Kits zum Durchführen eines Verfahrens nach einem der Ansprüche 1 bis 11, wobei der Kit umfasst: einen Primersatz für ein Gen, abgeleitet von einem oder mehreren Pathogenen, welche die Atemwege kolonisieren, ausgewählt aus der Gruppe bestehend aus Mikroben der Gattungen *Streptococcus, Haemophilus, Moraxella, Pseudomonas, Klebsiella, Stenotrophomonas, Acinetobacter* und *Staphylococcus;* und einer Anleitung.

13. Verwendung nach Anspruch 12, wobei der Kit weiterhin einen Primersatz für ein Gen, welches von einem oder mehreren Pathogenen abstammt, welche normalerweise in den Atemwegen abwesend sind, ausgewählt aus der Gruppe bestehend aus Mikroben der Gattungen *Mycoplasma, Legionella, Chlamydophila, Mycobacterium, Coxiella, Nocarida, Pneumocystis* und *Aspergillus;* und einer Anleitung.

14. Verwendung nach Anspruch 12 oder 13, wobei der Kit weiterhin umfasst:
einen Primersatz für ein oder mehrere Arzneimittel-resistente Gene ausgewählt aus der Gruppe bestehend aus einem mecA-Gen und einem metallo-β-lactamase-Gen; und einer Anleitung.

15. Verwendung einer Vorrichtung zum Durchführen eines Verfahrens nach einem der Ansprüche 1 bis 11, wobei die Vorrichtung umfasst:
a. DNA-Herstellungsmittel zum Herstellen von DNA aus einer Probe, welche die respiratorischen Sekrete eines Subjekts enthalten;
b. DNA-Vervielfältigungsmittel zum spezifischen Vervielfältigen von DNA, welche von Zellen des Subjekts abstammt, und DNA, das von Pathogen abstammt, in der DNA;
c. DNA-Detektionsmittel zum Detektieren der DNA, welche von Zellen des Subjekts abstammt, und der DNA, die von Pathogen abstammt, welche durch das DNA-Vervielfältigungsmittel vervielfältigt wird;
d. Berechnungsmittel zum Berechnen der relativen Anzahl einer Kopieanzahl der DNA, die von Pathogen abstammt, zu einer Kopieanzahl der DNA, welche von Zellen des Subjekts abgeleitet wird, basierend auf einem Signal, welches durch das DNA-Detektionsmittel detektiert wird; und
e. Erkennungsmittel zum Erkennen ob das Pathogen das respiratorische Pathogen ist oder nicht, basierend auf dem Erkennungsreferenzwert nach Anspruch 2 und der relativen Anzahl.

16. Verwendung nach Anspruch 15, wobei ein zusätzliches zu erkennendes Pathogen ein Pathogen ist, welches normalerweise in den Atemwegen abwesend ist, wobei die Vorrichtung weiterhin umfasst:
a. DNA-Herstellungsmittel zum Herstellen von DNA aus einer Probe, welche die respiratorischen Sekrete eines Subjekts enthalten;
b. DNA-Vervielfältigungsmittel zum spezifischen Vervielfältigen von DNA, die von Pathogen abstammt, in der DNA;
c. DNA-Detektionsmittel zum Detektieren der DNA, die von Pathogen abstammt, welche durch das DNA-Vervielfältigungsmittel vervielfältigt wird;
d. Erkennungsmittel zum Durchführen von Erkennung, so dass:
i. Wenn das Gen, das von Pathogen abstammt, nicht detektiert wird, das Pathogen nicht an der Infektion teilnimmt; und
ii. Wenn das Gen, das von Pathogen abstammt, detektiert wird, das Pathogen das respiratorische Pathogen ist.

## Revendications

1. Procédé permettant de distinguer un pathogène respiratoire provoquant une infection aiguë des voies respiratoires, dans lequel le pathogène devant être distingué comprend un pathogène colonisant les voies respiratoires, le procédé comprenant :
la mesure de la valeur relative de la quantité du pathogène dans un échantillon contenant les sécrétions respiratoires d'un sujet, par rapport à la quantité des cellules du sujet dans l'échantillon ; et
la distinction permettant de savoir si le pathogène est ou non le pathogène respiratoire, en se basant sur la valeur relative.

2. Procédé de détermination d'une valeur de référence de distinction permettant de distinguer un pathogène respiratoire provoquant une infection aiguë des voies respiratoires, dans lequel un pathogène devant être distingué est un pathogène colonisant les voies respiratoires, le procédé comprenant :
a) la mesure du nombre de copies d'un gène dérivé des cellules d'un sujet, dans un ADN préparé à partir d'un échantillon contenant les sécrétions respiratoires du sujet ;
b) la mesure du nombre de copies d'un gène dérivé du pathogène dans l'ADN ;
c) le calcul du nombre relatif du nombre de copies du gène dérivé du pathogène par rapport au nombre de copies du gène dérivé des cellules du sujet ;
d) la réalisation de a) à c) sur une pluralité de sujets afin d'obtenir le nombre relatif pour chacun des sujets ; et
e) la comparaison du nombre relatif de chacun des sujets à des résultats de diagnostic clinique de chacun des sujets afin de déterminer ainsi, comme valeur de référence de distinction :
i) la limite inférieure du nombre relatif menant à un diagnostic clinique positif de pathogène respiratoire ; et/ou
ii) la limite supérieure du nombre relatif menant à un diagnostic clinique négatif de pathogène respiratoire.

3. Procédé permettant de distinguer un pathogène respiratoire provoquant une infection aiguë des voies respiratoires, dans lequel un pathogène devant être distingué est un pathogène colonisant les voies respiratoires, le procédé comprenant :
a) la mesure du nombre de copies d'un gène dérivé des cellules d'un sujet, dans un ADN préparé à partir d'un échantillon contenant des sécrétions respiratoires du sujet ;
b) la mesure du nombre de copies d'un gène dérivé du pathogène dans l'ADN ;
c) le calcul du nombre relatif du nombre de copies du gène dérivé du pathogène par rapport au nombre de copies du gène dérivé des cellules du sujet ; et
d) la distinction permettant de savoir si le pathogène est ou non le pathogène respiratoire, en se basant sur la valeur de référence de distinction selon la revendication 2 et le nombre relatif.

4. Procédé selon la revendication 2 ou la revendication 3, dans lequel le pathogène colonisant les voies respiratoires est un ou plusieurs pathogènes choisis dans le groupe consistant en les microbes du genre *Streptococcus, Haemophilus, Moraxella, Pseudomonas, Klebsiella, Stenotrophomonas, Acinetobacter* et *Staphylococcus.*

5. Procédé selon la revendication 3 ou la revendication 4, dans lequel un pathogène supplémentaire devant être distingué est un pathogène qui est habituellement absent des voies respiratoires, le procédé comprenant en outre :
a) la mesure du nombre de copies d'un gène dérivé du pathogène dans un ADN préparé à partir d'un échantillon contenant les sécrétions respiratoires d'un sujet ; et
b) la réalisation de la distinction telle que :
i) lorsque le gène dérivé du pathogène n'est pas détecté, le pathogène ne participe pas à l'infection ; et
ii) lorsque le gène dérivé du pathogène est détecté, le pathogène est le pathogène respiratoire.

6. Procédé selon la revendication 5, dans lequel le pathogène qui est habituellement absent des voies respiratoires est un ou plusieurs pathogènes choisis dans le groupe consistant en des microbes du genre *Mycoplasma, Legionella, Chlamydophila, Mycobacterium, Coxiella, Nocardia, Pneumocystis* et *Aspergillus.*

7. Procédé selon l'une quelconque des revendications 2 à 6, dans lequel le gène dérivé du pathogène est un ou plusieurs gènes dérivés d'un pathogène colonisant les voies respiratoires, choisis dans le groupe consistant en un gène de pneumolysine *Streptococcus pneumonia,* un gène lytA de *Streptococcus pneumonia,* un gène d'ARNr 16S d'*Haemophilus influenza,* un gène copB de *Moraxella catarrhalis,* un gène d'ARNr 16S de *Pseudomonas,* un gène gapA de *Klebsiella pneumonia,* un gène d'ARNs 23S de *Stenotrophomonas maltophilia* et un gène femB de *Staphylococcus aureus.*

8. Procédé selon l'une quelconque des revendications 5 à 7, dans lequel le gène dérivé du pathogène est un ou plusieurs gènes dérivés d'un pathogène qui est habituellement absent des voies respiratoires, choisi dans le groupe consistant en le gène d'ARNr 16S de *Mycoplasma pneumonia,* un gène mip de *Legionella pneumophila,* un gène d'ARNr 16S de *Legionella,* un gène d'antigène 53-kD de *Chlamydophila pneumonia,* un gène ompA de *Chlamydophila psittaci,* un gène MPB64 de *Mycobacterium tuberculosis,* un gène d'ARNr ITS 16-23S de *Mycobacterium intracelulare,* un gène d'ARNr 16S de *Mycobacterium avium,* un gène d'ARNr ITS 16-23S de *Mycobacterium avium,* un gène dnaJ de *Mycobacterium kartsassi,* un gène d'ARNr 16S de *Nocardia,* et un gène d'ARNr 5S de *Pneumocystis jiroveci.*

9. Procédé selon l'une quelconque des revendications 2 à 8, dans lequel le gène dérivé du pathogène comprend en outre un gène de résistance au médicament.

10. Procédé selon la revendication 9, dans lequel le gène de résistance au médicament est un ou plusieurs gènes choisis dans le groupe consistant en un gène mecA et un gène métallo-β-lactamase.

11. Procédé selon l'une quelconque des revendications 1 à 10, dans lequel le sujet est un humain.

12. Utilisation d'un kit pour réaliser un procédé selon l'une quelconque des revendications 1 à 11, ledit kit comprenant : un jeu d'amorces pour un gène dérivé d'un ou plusieurs pathogènes colonisant les voies respiratoires, choisis dans le groupe consistant en des microbes du genre *Streptococcus, Haemophilus, Moraxella, Pseudomonas, Klebsiella, Stenotrophomonas, Acinetobacter,* et *Staphylococcus ;* et un manuel.

13. Utilisation selon la revendication 12, dans lequel le kit comprend en outre un jeu d'amorces pour un gène dérivé d'un ou plusieurs pathogènes qui sont habituellement absents des voies respiratoires, choisis dans le groupe consistant en les microbes du genre *Mycoplasma, Legionella, Chlamydophila, Mycobacterium, Coxiella, Nocardia, Pneumocystis,* et *Aspergillus ;* et un manuel.

14. Utilisation selon la revendication 12 ou la revendication 13, dans laquelle le kit comprend en outre : un jeu d'amorces pour un ou plusieurs gènes de résistance au médicament choisis dans le groupe consistant en un gène mecA et un gène métallo-β-lactamase ; et un manuel.

15. Utilisation d'un appareil pour réaliser un procédé selon l'une quelconque des revendications 1 à 11, l'appareil comprenant :
a) un moyen de préparation d'ADN destiné à préparer l'ADN à partir d'un échantillon contenant les sécrétions respiratoires d'un sujet ;
b) un moyen d'amplification d'ADN destiné à amplifier spécifiquement l'ADN dérivé des cellules du sujet et l'ADN dérivé du pathogène, dans l'ADN ;
c) un moyen de détection d'ADN destiné à détecter l'ADN dérivé des cellules du sujet et l'ADN dérivé du pathogène, amplifié par le moyen d'amplification d'ADN ;
d) un moyen de calcul destiné à calculer le nombre relatif d'un nombre de copies de l'ADN dérivé du pathogène par rapport à un nombre de copies de l'ADN dérivé des cellules du sujet, en se basant sur un signal détecté par le moyen de détection d'ADN ; et
e) un moyen de distinction destiné à distinguer si le pathogène est ou non le pathogène respiratoire, en se basant sur la valeur de référence de distinction selon la revendication 2 et le nombre relatif.

16. Utilisation selon la revendication 15, dans laquelle un pathogène supplémentaire devant être distingué est un pathogène qui est habituellement absent des voies respiratoires, l'appareil comprenant en outre :
